(19) Europäisches Patentamt
European Patent Office
Office européen des brevets

(11) EP 2 937 342 B1

(12) # EUROPÄISCHE PATENTSCHRIFT

(45) Veröffentlichungstag und Bekanntmachung des Hinweises auf die Patenterteilung:
**30.11.2016 Patentblatt 2016/48**

(51) Int Cl.:
***C07D 333/76*** *(2006.01)* ***C09K 19/34*** *(2006.01)*

(21) Anmeldenummer: **15000926.4**

(22) Anmeldetag: **30.03.2015**

(54) **4,6-DIFLUOR-DIBENZOTHIOPHEN-DERIVATE**

4,6-DIFLUORO DIBENZOTHIOPHENE DERIVATES

DÉRIVÉS DE 4,6-DIFLUORO-DIBENZOTHIOPHÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.04.2014 DE 102014005713**

(43) Veröffentlichungstag der Anmeldung:
**28.10.2015 Patentblatt 2015/44**

(73) Patentinhaber: **Merck Patent GmbH**
**64293 Darmstadt (DE)**

(72) Erfinder:
- **Reiffenrath, Volker**
**64380 Rossdorf (DE)**
- **Hirschmann, Harald**
**64291 Darmstadt (DE)**

(56) Entgegenhaltungen:
**WO-A1-02/055463 DE-A1-102005 012 585**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die vorliegende Erfindung betrifft 4,6-Difluor-dibenzothiophen-Derivate, ein Verfahren zu ihrer Herstellung, flüssigkristalline Medien enthaltend diese Derivate sowie elektrooptische Anzeigeelemente enthaltend diese flüssigkristallinen Medien. Die Verbindungen besitzen eine negative dielektrische Anisotropie.

Flüssigkristalle haben ein breites Anwendungsfeld gefunden, seitdem vor etwa 30 Jahren die ersten kommerziell anwendbaren flüssigkristallinen Verbindungen gefunden wurden. Bekannte Anwendungsgebiete für herkömmliche Mischungen sind insbesondere Anzeigen für Uhren und Taschenrechner sowie große Anzeigetafeln, wie sie in Bahnhöfen, Flughäfen und Sportarenen verwendet werden. Weitere Anwendungsgebiete sind Displays von tragbaren und stationären Computern, Navigationssystemen und Videoapplikationen. Insbesondere für die zuletzt genannten Anwendungen werden hohe Anforderungen an Schaltzeiten und den Kontrast der Abbildungen gestellt.

Die räumliche Ordnung der Moleküle in einem Flüssigkristall bewirkt, dass viele seiner Eigenschaften richtungsabhängig sind. Von Bedeutung für den Einsatz in Flüssigkristallanzeigen sind dabei insbesondere die Anisotropien im optischen, dielektrischen und elasto-mechanischen Verhalten. Je nachdem, ob die Moleküle mit ihren Längsachsen senkrecht oder parallel zu den beiden Platten eines Kondensators orientiert sind, hat dieser eine andere Kapazität; die Dielektrizitätskonstante $\varepsilon$ des flüssigkristallinen Mediums ist also für die beiden Orientierungen verschieden groß. Substanzen, deren Dielektrizitätskonstante bei senkrechter Orientierung der Moleküllängsachsen zu den Kondensatorplatten größer ist als bei paralleler Anordnung, werden als dielektrisch positiv bezeichnet. Mit anderen Worten: Ist die Dielektrizitätskonstante $\varepsilon\|$ parallel zu den Moleküllängsachsen größer als die Dielektrizitätskonstante $\varepsilon\perp$ senkrecht zu den Moleküllängsachsen, so ist die dielektrische Anisotropie $\Delta\varepsilon = \varepsilon\| - \varepsilon\perp$ größer null. Die meisten Flüssigkristalle, die in herkömmlichen Displays Verwendung finden, fallen in diese Gruppe.

Für die dielektrische Anisotropie spielen sowohl die Polarisierbarkeit des Moleküls als auch permanente Dipolmomente eine Rolle. Beim Anlegen einer Spannung an das Display richtet sich die Längsachse der Moleküle so aus, dass die größere der dielektrischen Konstanten wirksam wird. Die Stärke der Wechselwirkung mit dem elektrischen Feld hängt dabei von der Differenz der beiden Konstanten ab.

Bei den in herkömmlichen Flüssigkristallanzeigen verwendeten flüssigkristallinen Molekülen ist das entlang der Moleküllängsachse orientierte Dipolmoment größer als das senkrecht zur Moleküllängsachse orientierte Dipolmoment.

Mit Flüssigkristallen, bei denen das größere Dipolmoment parallel zur Längsachse des Moleküls orientiert ist, sind bereits sehr leistungsfähige Displays entwickelt worden. Dabei kommen meist Mischungen von 5 bis 20 Komponenten zum Einsatz, um einen ausreichend breiten Temperaturbereich der Mesophase sowie kurze Schaltzeiten und niedrige Schwellenspannungen anzustreben. Schwierigkeiten bereitet jedoch noch die starke Blickwinkelabhängigkeit bei Flüssigkristallanzeigen, wie sie beispielsweise für Laptops verwendet werden. Die beste Abbildungsqualität lässt sich erreichen, wenn die Fläche des Displays senkrecht zur Blickrichtung des Betrachters steht. Wird das Display relativ zur Betrachtungsrichtung gekippt, verschlechtert sich die Abbildungsqualität unter Umständen drastisch. Für einen höheren Komfort ist man bemüht, den Winkel, um den das Display von der Blickrichtung eines Betrachters ohne wesentliche Minderung der Abbildungsqualität verkippt werden kann, möglichst groß zu gestalten. In jüngerer Zeit sind Versuche unternommen worden, zur Verbesserung der Blickwinkelabhängigkeit flüssigkristalline Verbindungen einzusetzen, deren Dipolmoment senkrecht zur Moleküllängsachse größer ist als parallel zur Längsachse des Moleküls. Die dielektrische Anisotropie $\Delta\varepsilon$ ist in diesem Fall negativ. Im feldfreien Zustand werden diese Moleküle mit ihrer Längsachse senkrecht zur Glasfläche des Displays orientiert. Durch Anlegen eines elektrischen Feldes orientieren sie sich mehr oder weniger parallel zu den Glasflächen. Auf diese Weise konnte eine Verbesserung der Blickwinkelabhängigkeit erreicht werden. Derartige Displays werden als VA-TFT-Displays bezeichnet (abgeleitet aus dem Englischen: "vertically aligned").

Die Entwicklung auf dem Gebiet der flüssigkristallinen Materialien ist bei weitem noch nicht abgeschlossen. Zur Verbesserung der Eigenschaften flüssigkristalliner Anzeigeelemente ist man ständig bemüht, neue Verbindungen zu entwickeln, die eine Optimierung derartiger Displays ermöglichen.

In den Druckschriften WO 02/055463, DE 102005012585 und EP 1752510 werden Dibenzothiophenderivate für die Verwendung als flüssigkristallines Material offenbart. Die Verbindungen unterscheiden sich von den erfindungsgemäßen Verbindungen in der Substitution der Dibenzothiophenstruktur. Die Druckschriften offenbaren keine physikalischen Daten zu vergleichbaren Verbindungen.

Eine Aufgabe der vorliegenden Erfindung ist es, Verbindungen mit vorteilhaften Eigenschaften für den Einsatz in flüssigkristallinen Medien zur Verfügung zu stellen. Insbesondere sollten sie über eine negative dielektrische Anisotropie verfügen, was sie besonders geeignet macht für den Einsatz in flüssigkristallinen Medien für VA-Displays. Unabhängig von der dem Displaytyp entsprechenden dielektrischen Anisotropie sind Verbindungen gewünscht, die eine günstige Kombination der anwendungstechnischen Parameter aufweisen. Unter diesen gleichzeitig zu optimierenden Parametern sind vor allem zu nennen ein hoher Klärpunkt, eine geringe Rotationsviskosität, eine optische Anisotropie im Anwendungsintervall, sowie die Eigenschaften, die zur Erzielung von Mischungen mit den gewünschten flüssigkristallinen Phasen über einen breiten Temperaturbereich dienen (niedriger Schmelzpunkt, gute Mischbarkeit mit anderen flüssigkristallinen Komponenten der gewünschten Art).

Diese Aufgabe wird erfindungsgemäß gelöst durch Verbindungen der allgemeinen Formel I

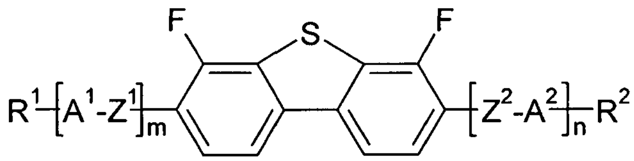

I

worin

m und n unabhängig voneinander 0, 1 oder 2 sind, bevorzugt 0,

$R^1$ und $R^2$ unabhängig voneinander einen Alkyl- oder Alkoxyrest mit 1 bis 15 C-Atomen, wobei in diesen Resten auch eine oder mehrere $CH_2$-Gruppen jeweils unabhängig voneinander durch $-C{\equiv}C-$, $-CF_2O-$, $-OCF_2-$, $-CH{=}CH-$,

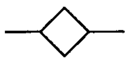 , 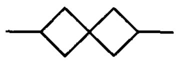 ,

-O-, -CO-O-, oder -O-CO- so ersetzt sein können, dass O-Atome nicht direkt miteinander verknüpft sind, und worin auch ein oder mehrere H-Atome durch Halogen ersetzt sein können, bevorzugt unabhängig voneinander, einen unsubstituierten Alkylrest oder Alkoxyrest mit 1 bis 15 Kohlenstoffatomen oder einen Alkenyl-, Alkenyloxy- oder Alkinylrest mit 2 bis 15 C-Atomen, welche jeweils optional ein- oder mehrfach halogeniert sind,

$A^1$ und $A^2$ unabhängig voneinander einen Rest ausgewählt aus folgenden Gruppen

a) 1,4-Phenylen worin auch eine oder zwei CH-Gruppen durch N ersetzt sein können und worin auch ein oder mehrere H-Atome durch eine Gruppe L ersetzt sein können

b) der Gruppe bestehend aus trans-1,4-Cyclohexylen und 1,4-Cyclohexenylen , worin auch eine oder mehrere nicht benachbarte $CH_2$-Gruppen durch -O- und/oder -S- ersetzt sein können und worin auch ein oder mehrere H-Atome durch F oder Cl ersetzt sein können, und

c) der Gruppe bestehend aus Tetrahydropyran-2,5-diyl, 1,3-Dioxan-2,5-diyl, Tetrahydrofuran-2,5-diyl, Cyclobutan-1,3-diyl, Piperidin-1,4-diyl, Thiophen-2,5-diyl und Selenophen-2,5-diyl, welche auch ein oder mehrfach durch ein Gruppe L substituiert sein können,

L bei jedem Auftreten unabhängig F, Cl, CN, SCN, $SF_5$ oder geradkettiges oder verzweigtes, jeweils optional fluoriertes, Alkyl, Alkoxy, Alkylcarbonyl, Alkoxycarbonyl, Alkylcarbonyloxy oder Alkoxycarbonyloxy mit 1 bis 12 oder vorzugsweise 1 bis 4 C-Atomen, und

$Z^1$ und $Z^2$ unabhängig voneinander eine Einfachbindung, $-CF_2O-$, $-OCF_2-$, $-CH_2O-$, $-OCH_2-$, -(CO)O-, -O(CO)-, $-(CH_2)_4-$, $-CH_2CH_2-$, $-CF_2-CF_2-$, $-CF_2-CH_2-$, $-CH_2-CF_2-$, $-CH{=}CH-$, $-CF{=}CF-$, $-CF{=}CH-$, $-CH{=}CF-$, $-(CH_2)_3O-$, $-O(CH_2)_3-$, $-C{\equiv}C-$, -O-, $-CH_2-$, $-(CH_2)_3-$ oder $-CF_2-$,

bedeuten.

Die Verbindungen besitzen ein ausgeprägt negatives $\Delta\varepsilon$ und eignen sich daher insbesondere für eine Verwendung in Flüssigkristallmischungen für VA-TFT-Displays. Vorzugsweise besitzen die erfindungsgemäßen Verbindungen ein $\Delta\varepsilon \leq -4$, mehr bevorzugt $\Delta\varepsilon \leq -6$ und besonders bevorzugt ein $\Delta\varepsilon \leq -8$. Sie zeigen eine gute Mischbarkeit mit den üblichen, in Flüssigkristallmischungen für Displays verwendeten Substanzen, d. h. sie besitzen eine gute Löslichkeit darin. Die Rotationsviskositäten der Verbindungen und der resultierenden flüssigkristallinen Mischungen sind vorteilhaft klein.

Auch die weiteren physikalischen, physikochemischen beziehungsweise elektrooptischen Parameter der erfindungsgemäßen Verbindungen sind für den Einsatz der Verbindungen in flüssigkristallinen Medien von Vorteil. Die

flüssigkristallinen Medien, die diese Verbindungen enthalten, weisen insbesondere eine ausreichende Breite der nematischen Phase und eine gute Tieftemperatur- und Langzeitstabilität sowie ausreichend hohe Klärpunkte auf. Die niedrigen Schmelzpunkte der erfindungsgemäßen Verbindungen geben einen Hinweis auf das vorteilhafte Mischungsverhalten. Ferner weisen die erfindungsgemäßen Verbindungen der Formel I insbesondere für die Verwendung in VA-TFT-Displays geeignete Werte der optischen Anisotropie $\Delta n$ auf. Bevorzugt besitzen die erfindungsgemäßen Verbindungen ein $\Delta n$ von größer als 0,15 und kleiner als 0,25. Außerdem sind die Verbindungen relativ einfach herzustellen. Die ausgewogene Kombination dieser vorteilhaften Eigenschaften stellt eine signifikante Bereicherung der für VA-Mischungen verfügbaren Mischungskomponenten dar.

Die Parameter m und n besitzen in der Summe m + n bevorzugt einen Wert von 0 oder 1, besonders 0. Somit ist m bevorzugt 0 oder 1, bevorzugt 0, und n bevorzugt 0.

Bevorzugt bedeuten $R^1$ und $R^2$ jeweils unabhängig voneinander einen Alkoxyrest, Alkylrest oder Alkenylrest mit 1 bis 7 bzw. 2 bis 7 Kohlenstoffatomen. Besonders bevorzugt sind $R^1$ und $R^2$ unabhängig voneinander in der allgemeinen Formel I ein Alkoxyrest oder Alkylrest mit 2 bis 7 C-Atomen. Dabei sind die Reste $R^1$ und $R^2$ bevorzugt verschieden.

Für den Fall, dass m = 0 ist, bedeutet $R^1$ vorzugsweise eine Alkoxy-, Alkyl- oder Alkenylgruppe, besonders bevorzugt eine Alkoxygruppe mit 1-7 C-Atomen, besonders bevorzugt mit 2 bis 5 C-Atomen. Für den Fall, dass n = 0 ist, bedeutet $R^2$ vorzugsweise eine Alkoxy-, Alkyl- oder Alkenylgruppe, besonders bevorzugt eine Alkoxygruppe mit 1-7 C-Atomen, besonders bevorzugt mit 2 bis 7 C-Atomen. Die Summe der Anzahl der Kohlenstoffatome in $R^1$ und $R^2$ zusammen beträgt bevorzugt 4, 5, 6, 7, 8, 9 oder 10, besonders bevorzugt 6, 7, 8, 9 oder 10.

Für den Fall, dass m = 1 oder 2 ist, bedeutet $R^1$ vorzugsweise eine Alkyl-, Alkoxy- oder Alkenylgruppe, besonders bevorzugt eine Alkylgruppe mit 1-7 C-Atomen, besonders bevorzugt mit 2 bis 5 C-Atomen. Für den Fall, dass n = 1 oder 2 ist, bedeutet $R^2$ vorzugsweise eine Alkyl- oder Alkenylgruppe, besonders bevorzugt eine Alkylgruppe mit 1-7 C-Atomen, besonders bevorzugt mit 2 bis 5 C-Atomen.

Sofern $R^1$ und $R^2$ in Formel I jeweils unabhängig voneinander einen Alkylrest darstellen, sind diese geradkettig oder verzweigt. Vorzugsweise ist jeder dieser Reste geradkettig, und hat soweit nicht anders angegeben 1, 2, 3, 4, 5, 6 oder 7 C-Atome und ist demnach vorzugsweise Methyl, Ethyl, Propyl, Butyl, Pentyl, Hexyl oder Heptyl.

Sofern $R^1$ und $R^2$ in Formel I jeweils unabhängig voneinander einen Alkoxyrest darstellen, sind diese geradkettig oder verzweigt. Vorzugsweise ist jeder dieser Reste geradkettig, und hat soweit nicht anders angegeben 1, 2, 3, 4, 5, 6 oder 7 C-Atome und ist demnach vorzugsweise Methoxy, Ethoxy, Propoxy, Butoxy, Pentoxy, Hexoxy oder Heptoxy.

$R^1$ und $R^2$ in Formel I können ferner jeweils unabhängig voneinander ein Alkenylrest mit 2 bis 15 C-Atomen sein, der geradkettig oder verzweigt ist und wenigstens eine C-C-Doppelbindung aufweist. Vorzugsweise ist er geradkettig und hat 2 bis 7 C-Atome. Er ist demnach vorzugsweise Vinyl, Prop-1- oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl. Sind die beiden C-Atome der C-C-Doppelbindung substituiert, kann der Alkenylrest als E- und/oder Z-Isomer (trans/cis) vorliegen. Im Allgemeinen sind die jeweiligen E-Isomere bevorzugt. Unter den Alkenylresten sind besonders bevorzugt Prop-2-enyl, 2- oder But-3-enyl, und 3- oder Pent-4-enyl.

$R^1$ und $R^2$ in Formel I können unabhängig voneinander auch ein Alkinylrest mit 2 bis 15 C-Atomen sein, der geradkettig oder verzweigt ist und wenigstens eine C-C-Dreifachbindung aufweist. Bevorzugt ist 1- oder 2 Propinyl und 1-, 2- oder 3- Propinyl.

Die Gruppen $A^1$ und $A^2$ bedeuten unabhängig bevorzugt eine disubstituierte ringförmige Gruppe ausgewählt aus den Formeln

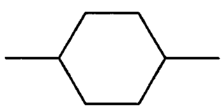 , 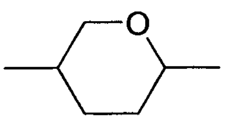 , 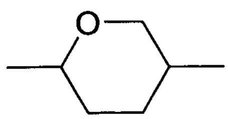 , 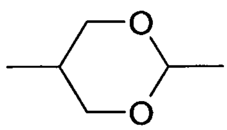 ,

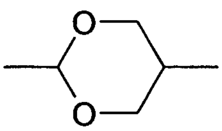 , 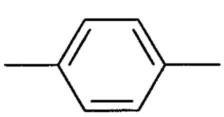 , 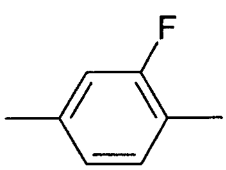 , 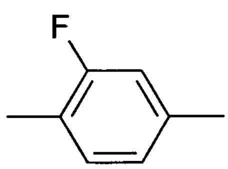

oder 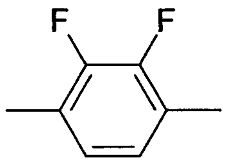 ,

insbesondere

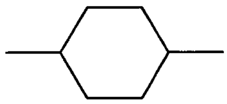 , 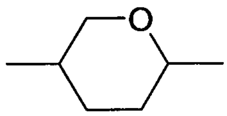 ,

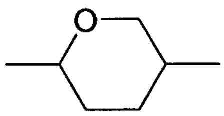 , 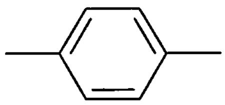 , 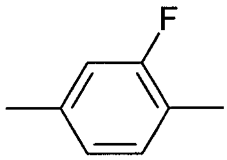 , 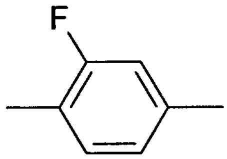

oder 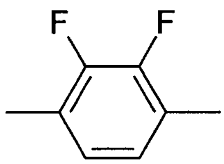 .

Die Gruppe $Z^1$ bedeutet bevorzugt eine Einfachbindung, $-CH_2O-$, $-CF_2O-$ oder $-OCF_2-$, besonders bevorzugt eine Einfachbindung.

Die Gruppe $Z^2$ bedeutet bevorzugt eine Einfachbindung, $-OCH_2-$, $-OCF_2-$ oder $-CF_2O-$, besonders bevorzugt eine Einfachbindung.

Die Gruppe L bedeutet bevorzugt F, Cl, $-CF_3$ oder eine Alkyl- oder Alkoxygruppe mit 1, 2 oder 3 Kohlenstoffatomen.

Besonders bevorzugt bedeuten m und n 0 und $R^1$ und $R^2$ jeweils eine Alkoxygruppe mit 1 bis 7 Kohlenstoffatomen.

Halogen bedeutet im Zusammenhang der vorliegenden Erfindung Fluor, Chlor, Brom und Iod, insbesondere Fluor oder Chlor.

Im Zusammenhang mit der vorliegenden Erfindung bedeutet der Ausdruck "Alkyl" - sofern er nicht an anderer Stelle dieser Beschreibung oder in den Ansprüchen abweichend definiert ist - einen geradkettigen oder verzweigten, gesättigten, aliphatischen Kohlenwasserstoffrest mit 1 bis 15 (d.h. 1, 2, 3, 4, 5, 6, 7, 8, 9, 10, 11, 12, 13, 14 oder 15) Kohlenstoffatomen.

Besonders bevorzugt sind erfindungsgemäße Verbindungen der Formel I ausgewählt aus den Unterformeln IA bis IC

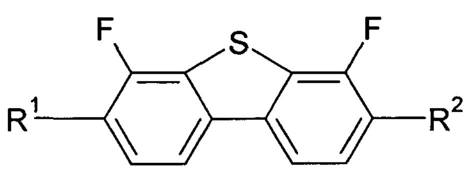 IA

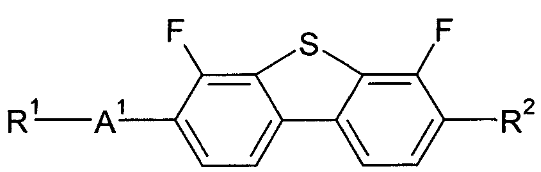 IB

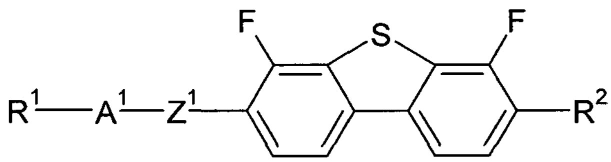

IC

worin $R^1$, $R^2$ und $A^1$ die Bedeutungen wie für die Formel I oben definiert besitzen, und $Z^1$ eine Gruppe wie oben definiert ohne der Einfachbindung ist.

Bevorzugte Verbindungen der Formel IA sind die Verbindungen der Formeln IA-1 bis IA-10,

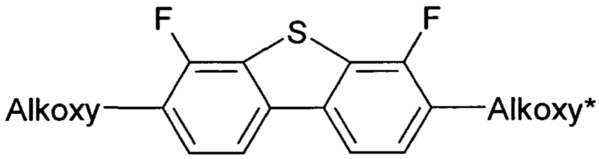

IA-1

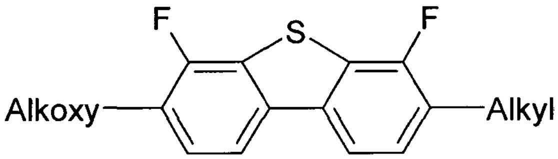

IA-2

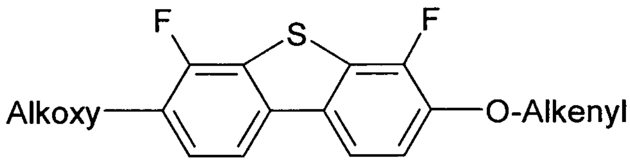

IA-3

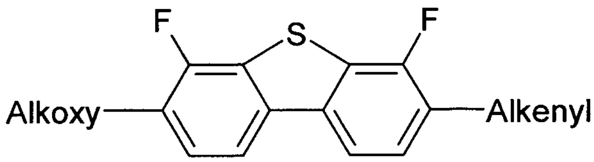

IA-4

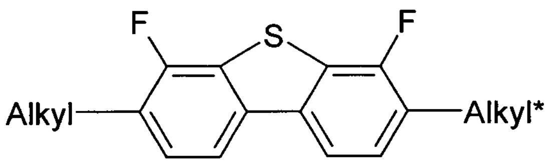

IA-5

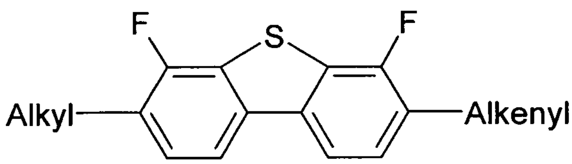

IA-6

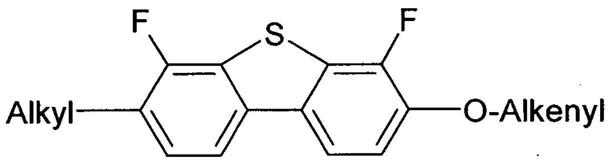

IA-7

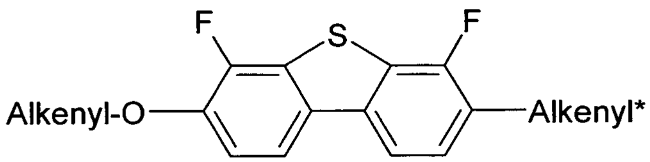

IA-8

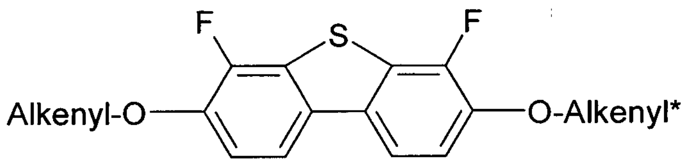

IA-9

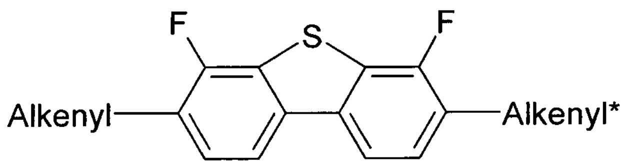

IA-10,

worin Alkyl und Alkyl* jeweils unabhängig voneinander einen geradkettigen Alkylrest mit 1-7 C-Atomen, Alkenyl und Alkenyl* jeweils unabhängig voneinander einen geradkettigen Alkenylrest mit 2-7 C-Atomen, Alkoxy und Alkoxy* jeweils unabhängig voneinander einen geradkettigen Alkoxyrest mit 1-7 C-Atomen bedeuten. Besonders bevorzugt sind die Verbindungen der Formeln IA-1, IA-2, IA-3, IA-4, IA-7 und IA-9.

Bevorzugte Verbindungen der Formeln IB und IC sind die Verbindungen der Formeln,

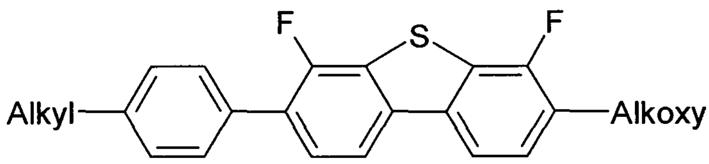

IB-1

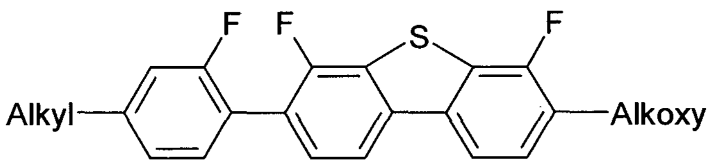

IB-2

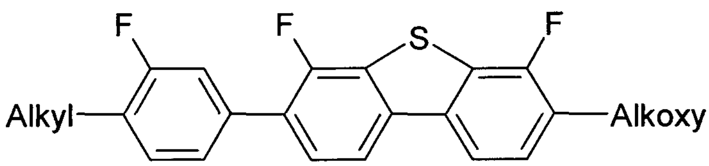

IB-3

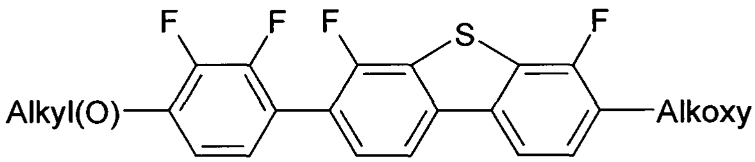

IB-4

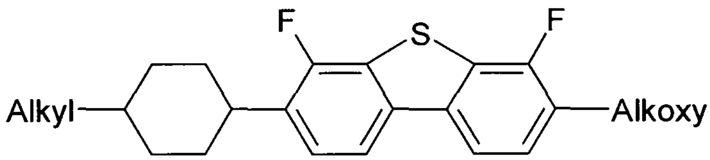

IB-5

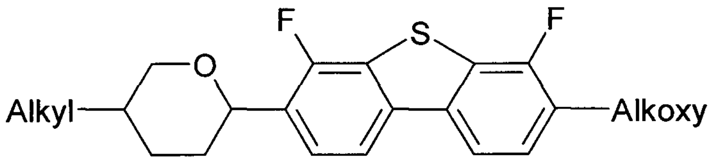

IB-6

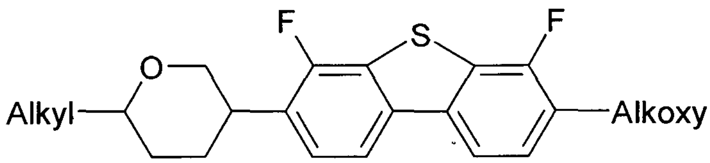

IB-7

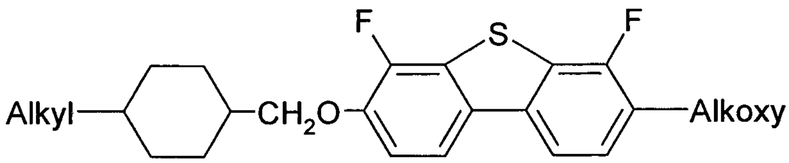

IC-1

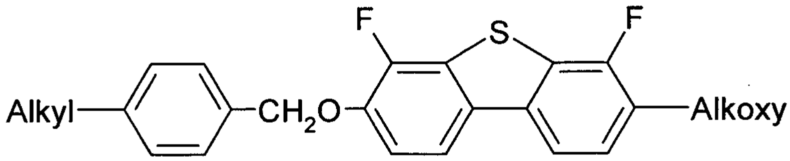

IC-2

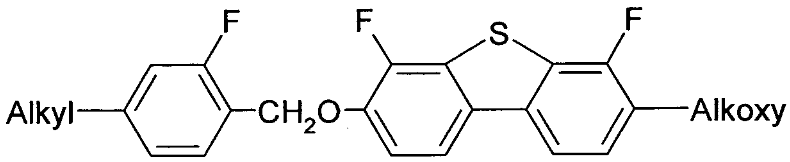

IC-3

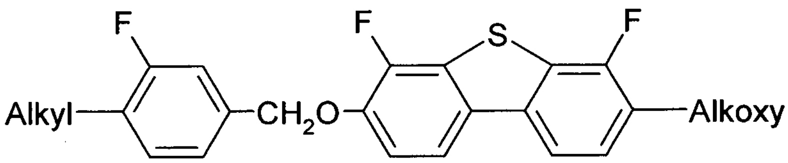

IC-4

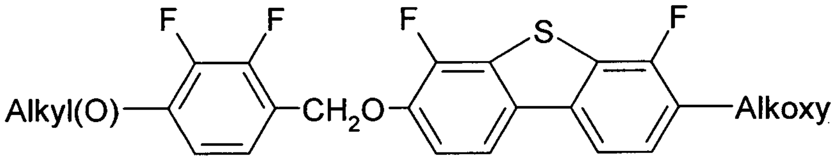

IC-5

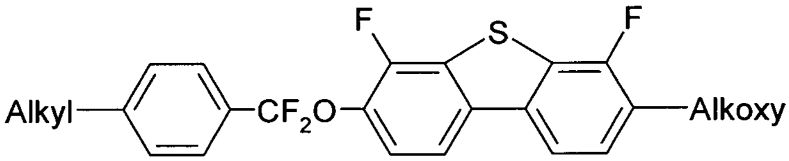

IC-6

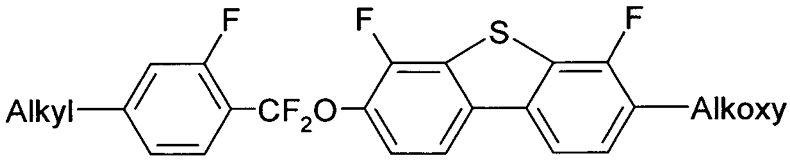

IC-7

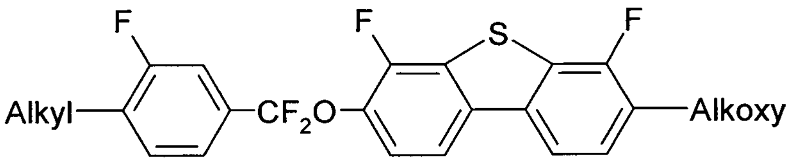

IC-8

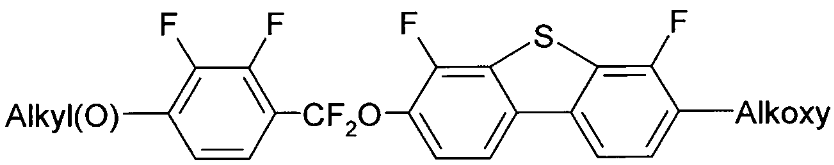

IC-9

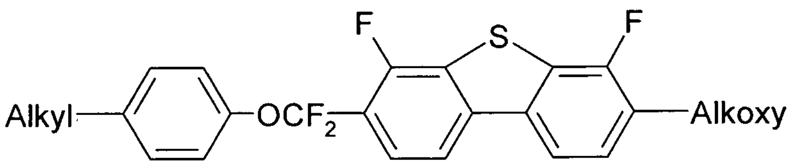

IC-10

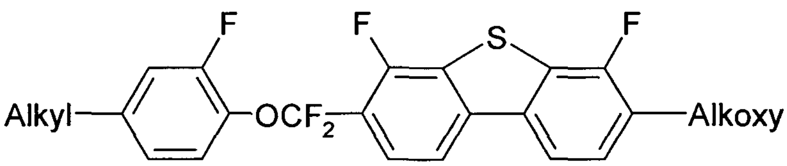

IC-11

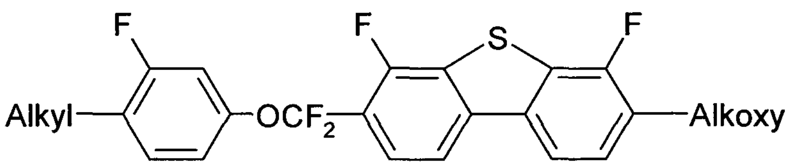

IC-12

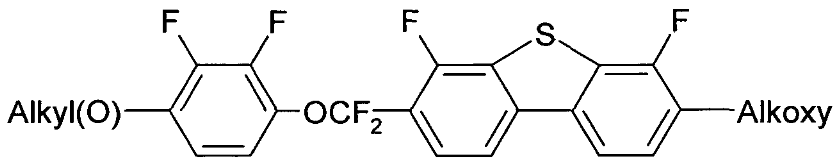 IC-13

worin Alkyl unabhängig voneinander einen geradkettigen Alkylrest mit 1-7 C-Atomen bedeutet. Der Rest Alkyl(O) steht für Alkyl- oder Alkoxy mit 1 bis 7 C-Atomen. Besonders bevorzugt sind darunter die Verbindungen der Formeln IB-5 bis IC-13.

Bevorzugte Verbindungen der Formel I bzw. IA-1 sind ausgewählt aus denen der Formeln IA-1-1 bis IA-1-25:

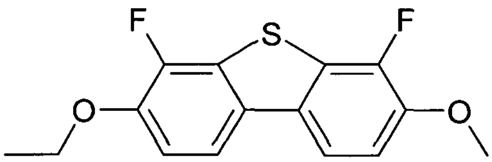

IA-1-1

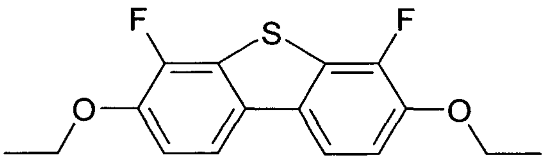

IA-1-2

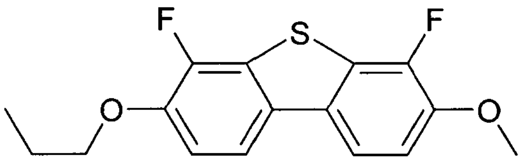

IA-1-4

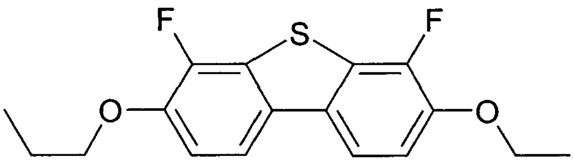

IA-1-5

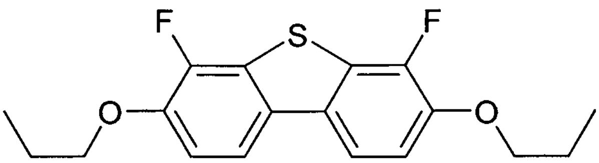

IA-1-6

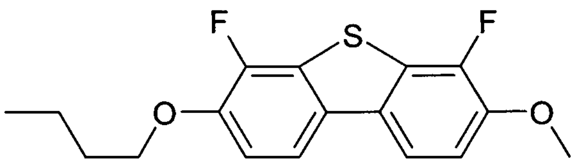

IA-1-7

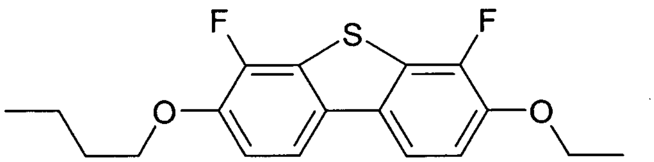

IA-1-8

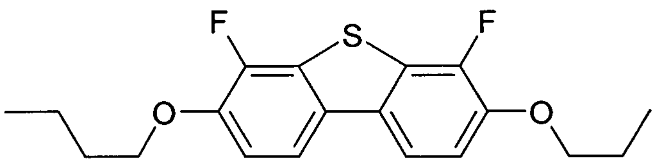

IA-1-9

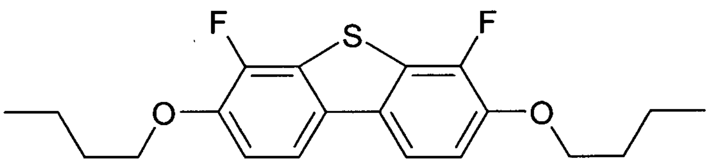

IA-1-10

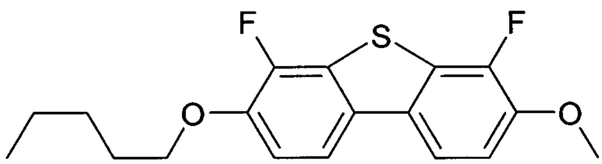

IA-1-11

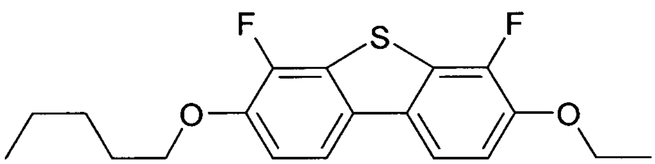

IA-1-12

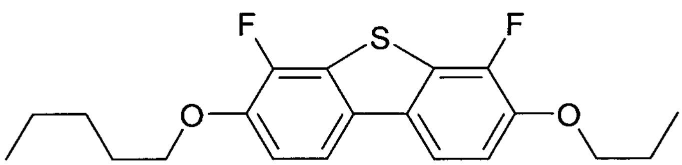

IA-1-13

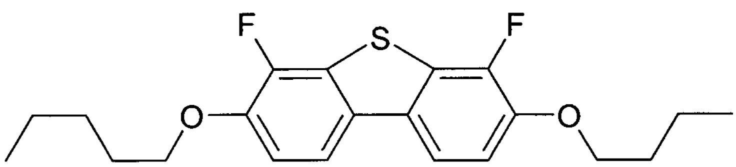

IA-1-14

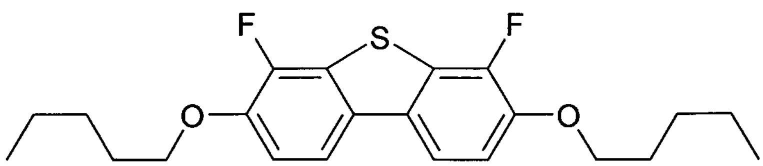

IA-1-15

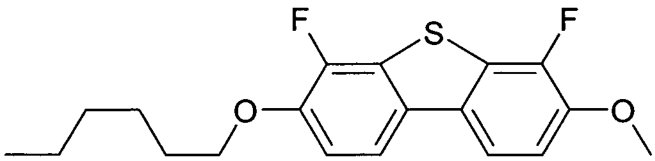

IA-1-16

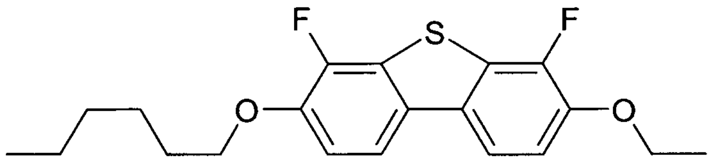

IA-1-17

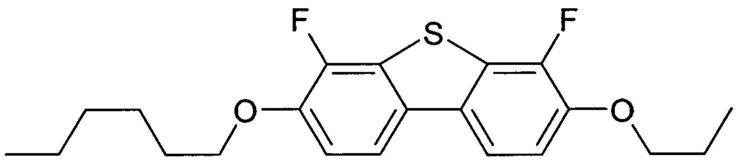

IA-1-18

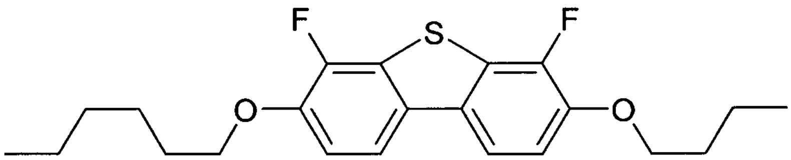

IA-1-19

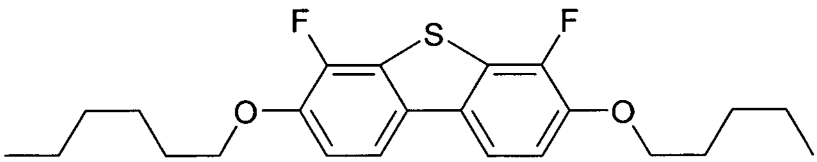

IA-1-20

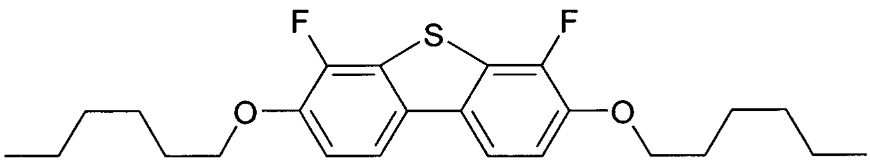

IA-1-21

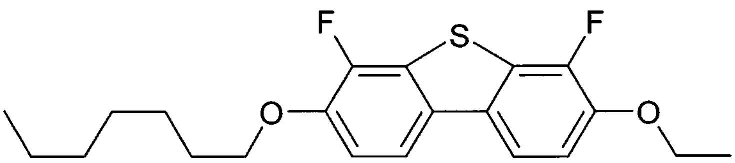

IA-1-22

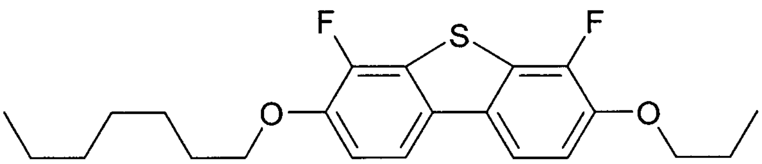

IA-1-23

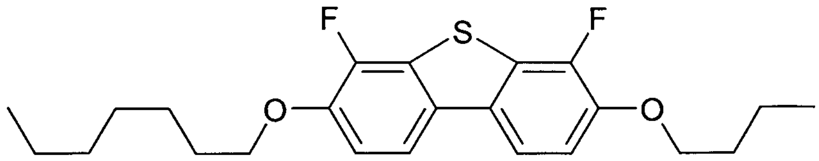

IA-1-24

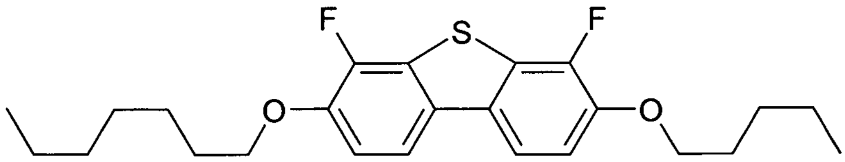

IA-1-25

insbesondere der Formeln IA-1-8, IA-1-12, IA-1-13, IA-1-17, IA-1-18, IA-1-19, IA-1-23 und IA-1-24".

Bevorzugte Verbindungen der Formel IA-2 sind ausgewählt aus den Folgenden:

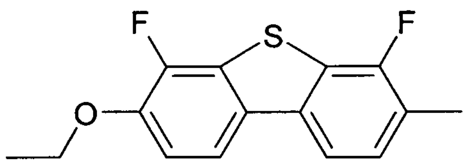

IA-2-1

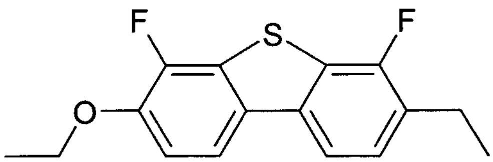

IA-2-2

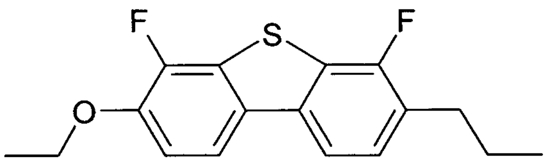

IA-2-3

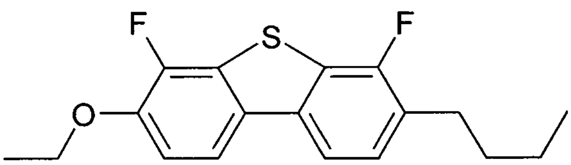

IA-2-4

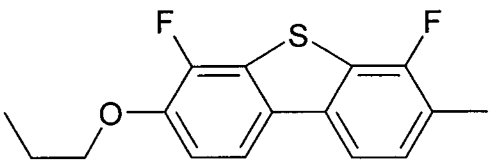

IA-2-5

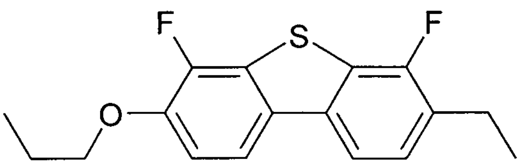

IA-2-6

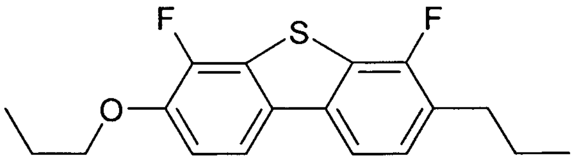

IA-2-7

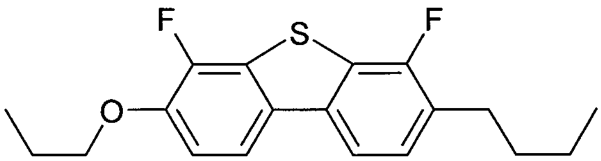

IA-2-8

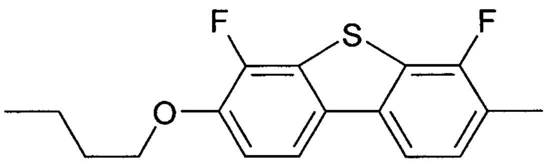

IA-2-9

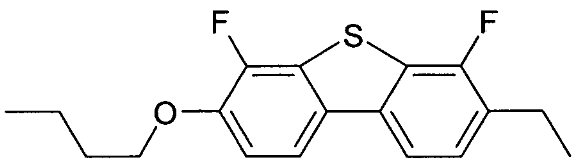

IA-2-10

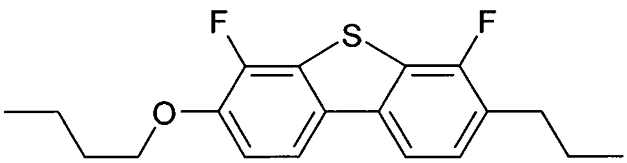

IA-2-11

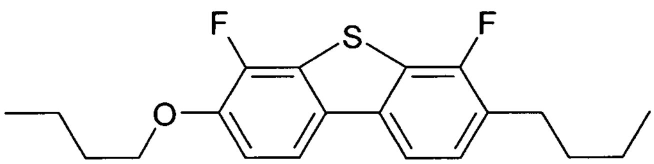

IA-2-12

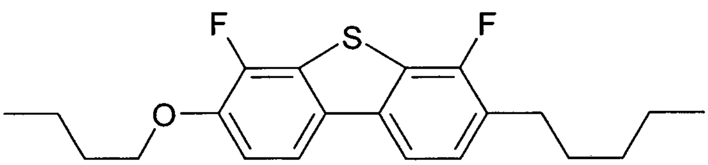

IA-2-13

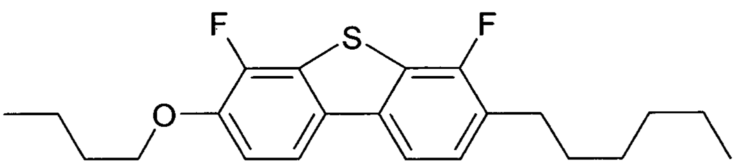

IA-2-14

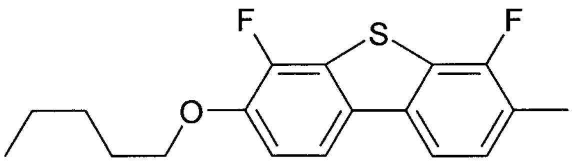

IA-2-15

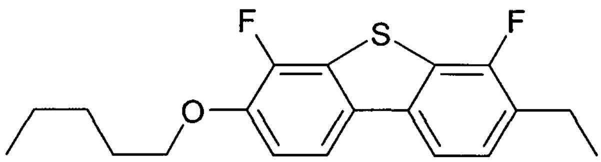

IA-2-16

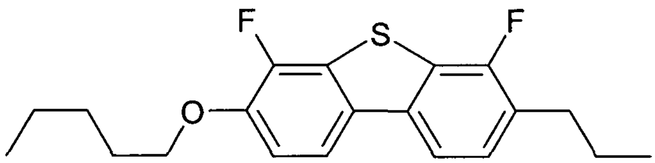

IA-2-17

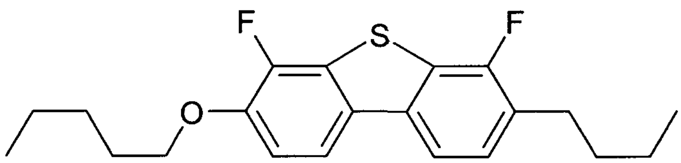

IA-2-18

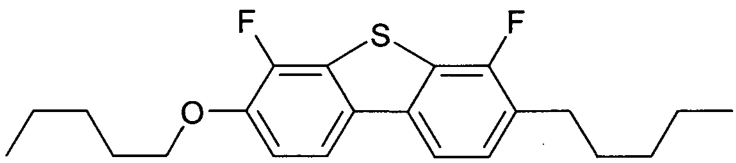

IA-2-19

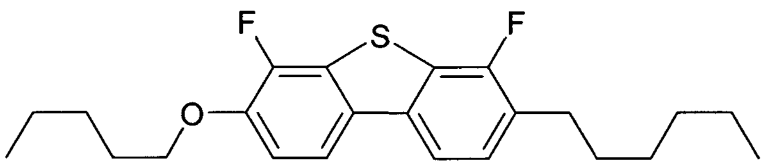

IA-2-20

Darunter sind besonders bevorzugt die Verbindungen der Formeln IA-2-7, IA-2-8, IA-2-12, IA-2-13, IA-2-16, IA-2-17 und IA-2-18.

Sofern Reste oder Substituenten der erfindungsgemäßen Verbindungen beziehungsweise die erfindungsgemäßen Verbindungen selbst als optisch aktive oder stereoisomere Reste, Substituenten beziehungsweise Verbindungen vorliegen, weil sie beispielsweise ein asymmetrisches Zentrum aufweisen, so sind diese von der vorliegenden Erfindung mit umfasst. Dabei ist es selbstverständlich, dass die erfindungsgemäßen Verbindungen der allgemeinen Formel I in isomerenreiner Form, zum Beispiel als reine Enantiomeren, Diastereomeren, E- beziehungsweise Z-Isomeren, trans- beziehungsweise cis-Isomeren, oder als Gemisch mehrerer Isomeren in jedem beliebigen Verhältnis, zum Beispiel als Racemat, E-/Z-Isomerengemisch oder als cis/trans-Isomerengemisch, vorliegen können.

Der 1,4-substituierte Cyclohexylring der Formel

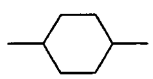

oder -Cycist in den offenbarten Verbindungen für flüssigkristalline Medien bevorzugt trans-konfiguriert, d.h. die beiden

Substituenten befindend sich in der thermodynamisch bevorzugten Sesselkonformation beide in äquatorialer Position.

Die Verbindungen der allgemeinen Formel I können nach an sich bekannten Methoden dargestellt werden, wie sie in der Literatur (z.B. in den Standardwerken wie Houben-Weyl, Methoden der organischen Chemie, Georg-Thieme-Verlag, Stuttgart) beschrieben sind und zwar unter Reaktionsbedingungen, die für die genannten Umsetzungen bekannt und geeignet sind. Dabei kann man von an sich bekannten, hier nicht näher erwähnten Varianten Gebrauch machen.

Die Ausgangsstoffe können gegebenenfalls auch *in situ* gebildet werden, derart, dass man sie aus dem Reaktionsgemisch nicht isoliert, sondern sofort weiter zu den Verbindungen der allgemeinen Formel I umsetzt.

Die Synthesen erfindungsgemäßer Verbindungen der allgemeinen Formel I werden in den Beispielen exemplarisch beschrieben. Die Ausgangssubstanzen sind nach allgemein zugänglichen Literaturvorschriften oder käuflich zu erhalten.

Besonders geeignete Synthesewege zu den erfindungsgemäßen Verbindungen werden im Folgenden anhand der Schemata 1 bis 6 erläutert. Die Substituenten $R^1$, $R^2$ und die Zähler m und n besitzen in den folgenden Schemata die Bedeutungen wie für die Formel I angegeben.

Die Synthese der Verbindungen der Formel I mit zwei Alkoxygruppen ($R^1$, $R^2$) und m/n = 0 erfolgt ausgehend von der Grundverbindung Dibenzothiophen (vgl. Schema 1).

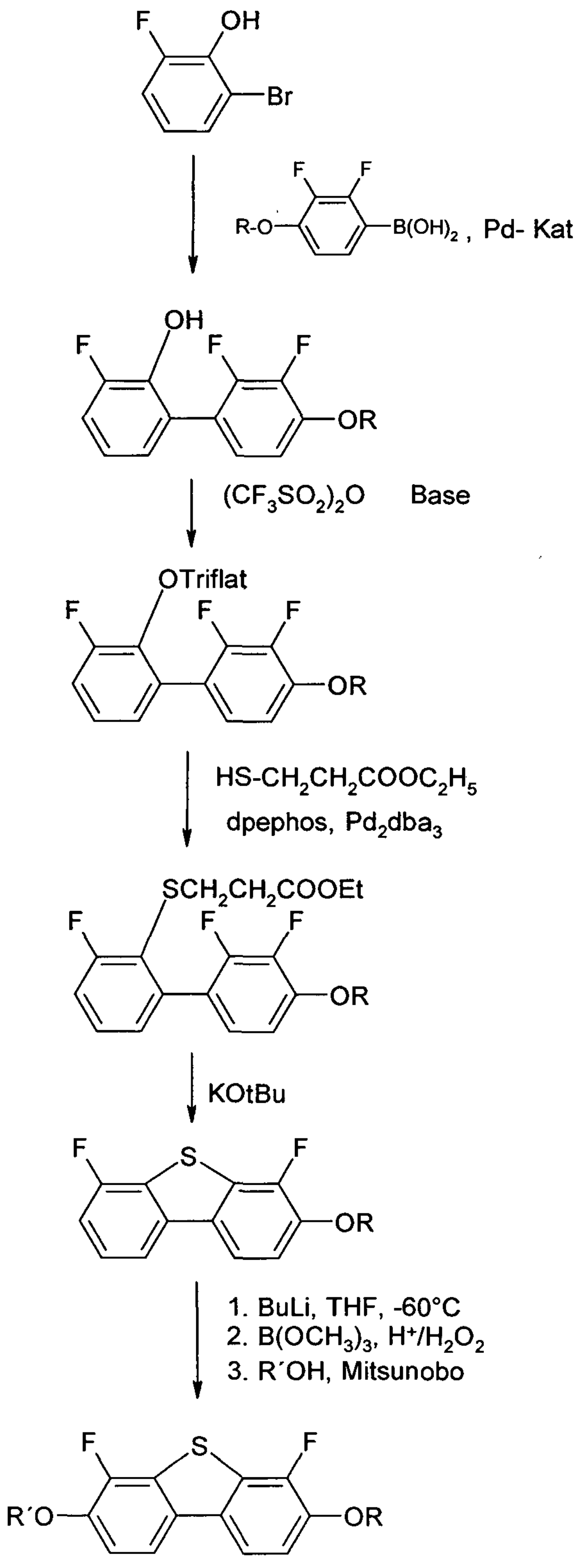


**Schema 1**. Synthese der Verbindungen der Formel I. Die Reste -R, -R' bedeuten jeweils unabhängig voneinander geradkettiges oder verzweigtes Alkyl oder Alkenyl analog Formel I. Das Mitsunobu-Protokoll benutzt die üblichen Reagenzien $PPh_3$ und ROOC-N=N-COOR, alternativ auch R-Br mit $K_2CO_3$.

Die Verbindungen mit einer Alkoxygruppe und einer Alkylgruppe (m + n = 0) werden abgewandelt von der

obigen Synthese gemäß dem Schema 2 hergestellt.

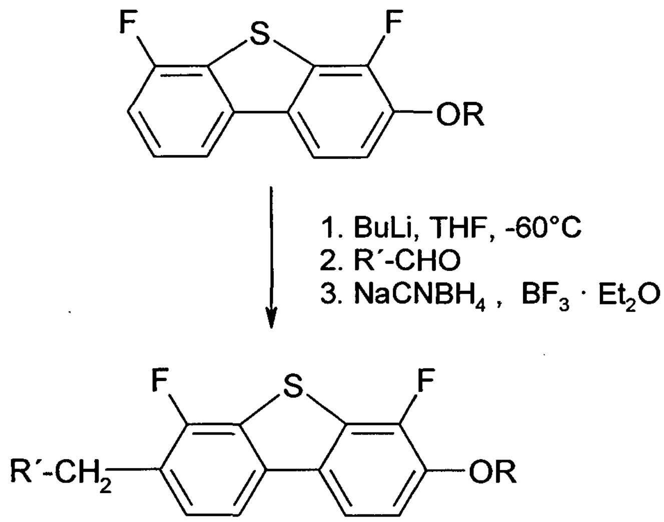


**Schema 2**. Synthese der Verbindungen der Formel I mit Alkyl- und Alkoxygruppe (m + n = 0). Der Rest R' entspricht einem entsprechend um ein Kohlenstoffatom verkürzten Rest wie $R^1/R^2$ in Formel I. Rest R siehe Schema 1.

Die Synthese der Verbindungen der Formel I mit zwei Alkylgruppen (m + n = 0) erfolgt in einer weiteren Abwandlung der obigen Synthesen (Schema 3).

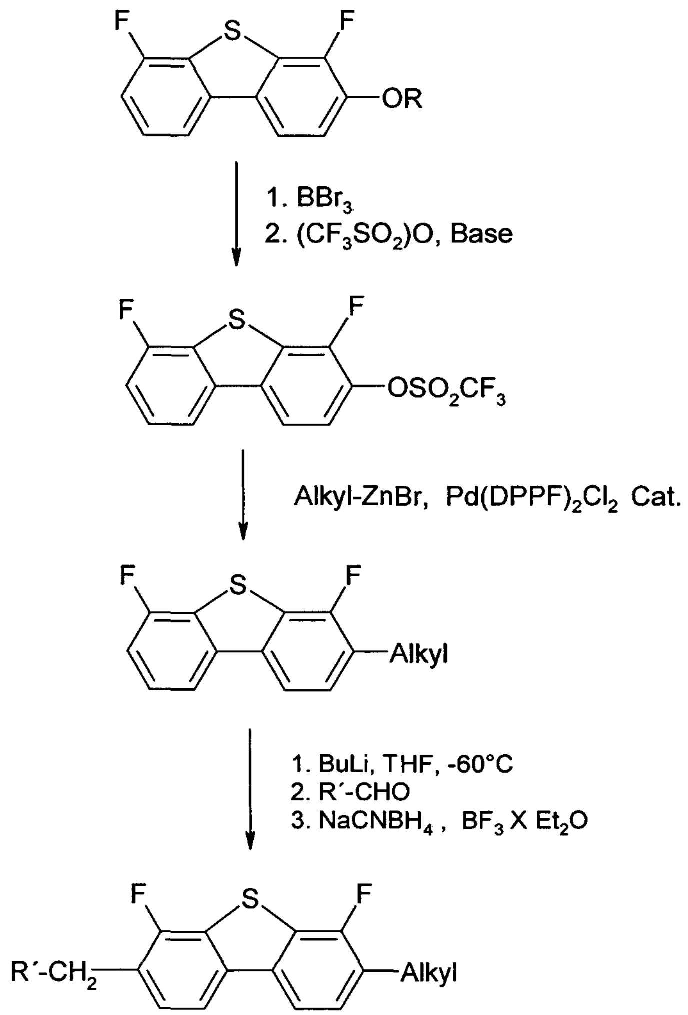


**Schema 3**. Synthese der Verbindungen der Formel I mit zwei Alkylgruppen (m + n = 0). Der Rest R' des eingesetzten Aldehyds entspricht einem entsprechend um ein Kohlenstoffatom verkürzten Rest wie $R^1$/$R^2$ der Formel I. Die Startverbindung kann wie in Schema 1 erhalten werden.

Die Startverbindung (Zugang vgl. Schema 1) wird in Schema 3 an der Etherfunktion gespalten (z.B. mit $BBr_3$). Anschließend wird die OH-Gruppe mit Trifluormethansulfonsäure verestert und anschließend einer Pdkatalysierten Kopplungsreaktion mit einer organischen Zink-Halogenverbindung (hier $R^1$-ZnBr) unterworfen. Die weiteren Schritte zur Erzeugung der zweiten Alkylgruppe entsprechen denen aus Schema 2.

Die Verbindungen der Formel I, worin weitere Ringe $A^{1/2}$ umfasst sind (m + n = 1, 2), werden beispielsweise gemäß Schemata 4 bis 6 hergestellt:

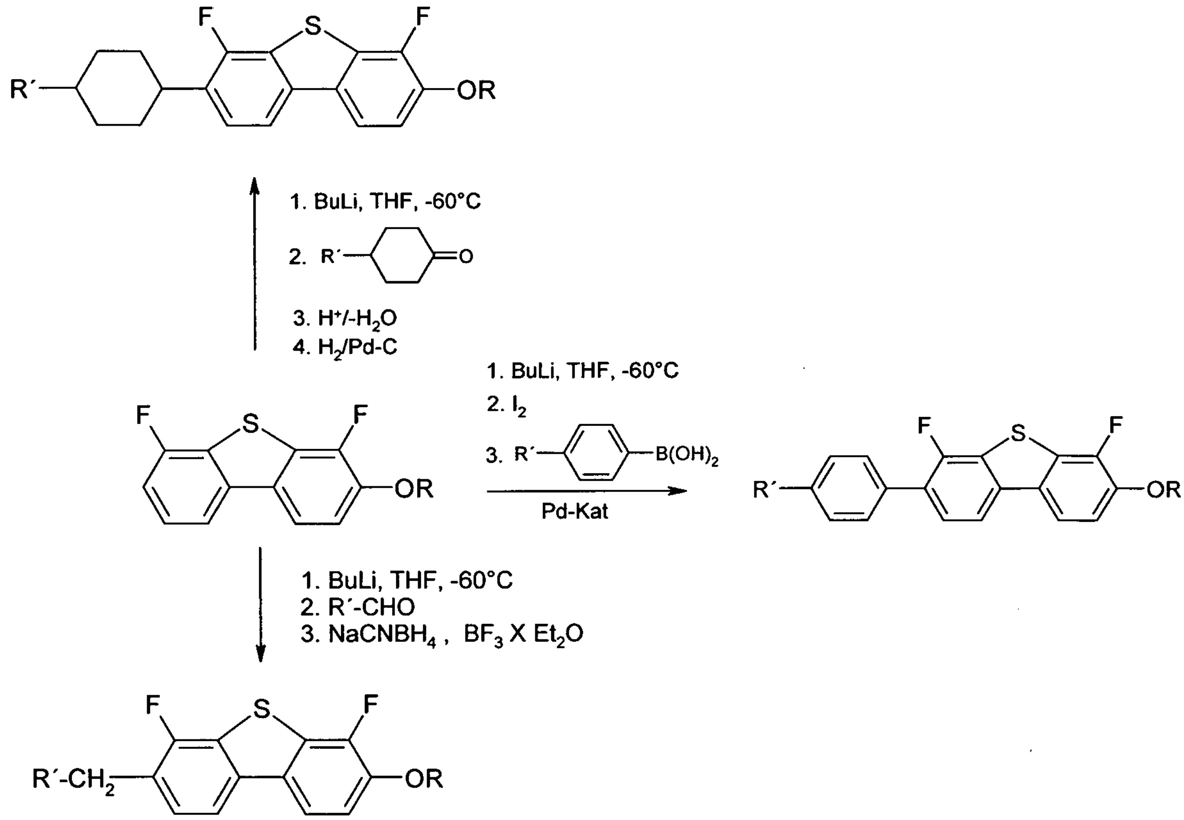


**Schema 4**. Synthese von Verbindungen der Formel I worin m = 1 und $Z^1$ eine Einfachbindung ist. Reste R und R' vgl. Schema 2, $R^2$ auch $-CH_2A^2$-$R^2$.

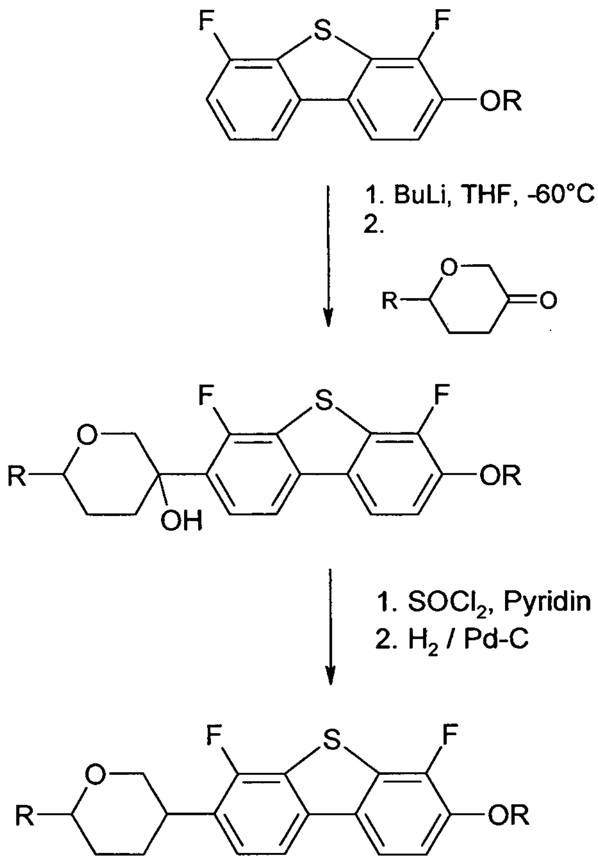


**Schema 5**. Synthese der Verbindungen der Formel I mit m = 1, $A^1$ = Tetrahydropyran und $Z^1$ eine Einfachbindung. Reste R und R' siehe Schema 1.

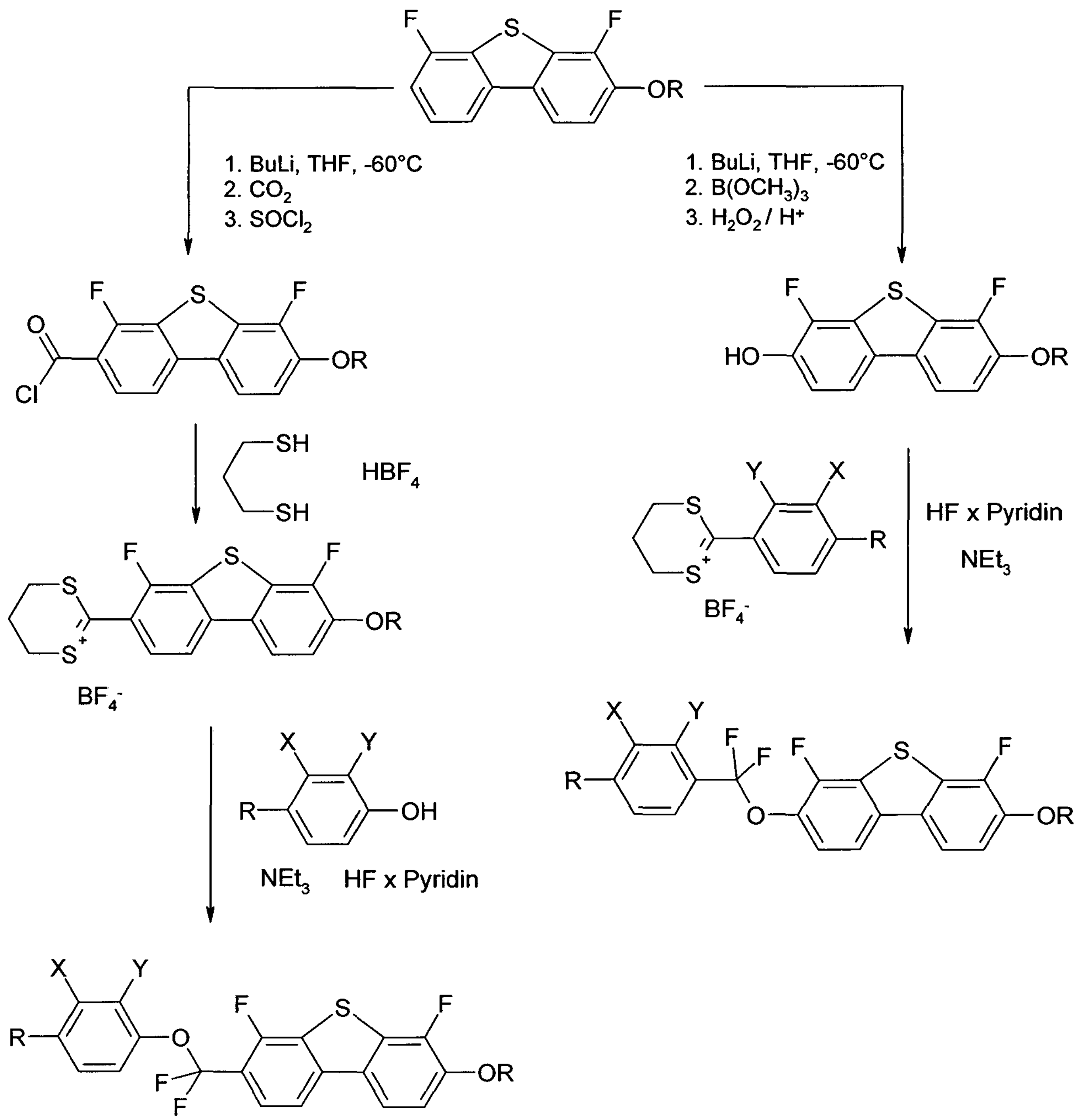


**Schema 6**. Synthese der Verbindungen der Formel I worin m = 1 und $Z^1$ = $-OCF_2-$ oder $-CF_2O$ -. Reste R und R' siehe Schema 1.

Prinzipiell sind die Synthesen von Alkoxyresten (hier-OR) analog auf andere Etherverbindungen, z.B. $-OCH_2A^1-R^1$ bzw. $-OCH_2A^2-R^2$ übertragbar.

Die dargestellten Reaktionsschemata sind nur als beispielhaft aufzufassen. Optional können anstelle der Reste -OR und -OR' in den Schemata auch allgemeiner Reste der Formeln $R^1-[A^1-Z^1]_m-$ und $-[Z^2-A^2]_n-R^2$ analog der allgemeinen Formel I eingeführt werden. Der Fachmann kann entsprechende Variationen der vorgestellten Synthesen vornehmen, sowie auch andere geeignete Synthesewege beschreiten, um Verbindungen der Formel I zu erhalten.

Gemäß den zuvor dargestellten Synthesen umfasst die vorliegende Erfindung in einer Ausführungsform auch ein oder mehrere Verfahren zur Herstellung von Verbindungen der Formel I.

Die Erfindung umfasst somit ein Verfahren zur Herstellung von Verbindungen der Formel I, das dadurch gekennzeichnet ist, dass es einen Verfahrensschritt umfasst, wobei eine Verbindung der Formel (B)

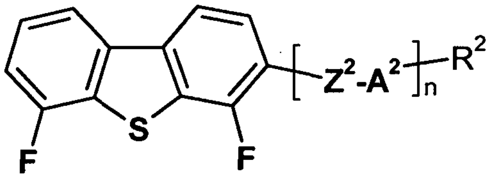

(B)

worin n, $Z^2$, $A^2$, $R^2$ unabhängig wie in Formel I definiert sind, an der Position 3 mittels eines Deprotonierungsreagenzes deprotoniert und zu einer Verbindung der Formel (C)

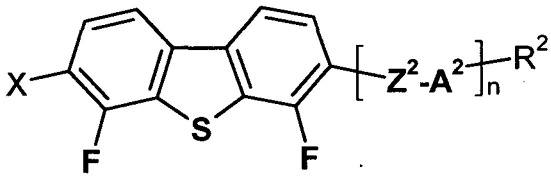

(C)

worin unabhängig

X $B(OR)_2$, $-C(OH)R_2$, -(CO)OH, -(CO)Cl, OH oder

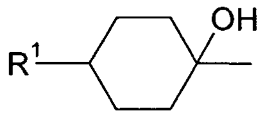

bedeutet,
R jeweils unabhängig H oder einen Alkylrest mit 1 bis 14 C-Atomen bedeutet, und
$Z^2$, $A^2$, n, $R^1$, $R^2$ wie in Formel I definiert sind, umgesetzt wird, und in weiteren Verfahrensschritten zu einer Verbindung der Formel I umgesetzt wird.

Die verschiedenen Gruppen X in Formel (C) werden erhalten, indem man den in *ortho*-Position zum Fluoratom metallierten Aromaten mit Borsäuretrialkylester $B(OR)_3$ zu X= $-B(OR)_2$, mit Aldehyd RCHO zu -C(OH)R, 4-($R^1$)-substituierte Cyclohexanone zu den entsprechenden Alkoholen und ggf. die entstandene Boronsäuregruppe X= $-B(OR)_2$ oxidativ (z.B. mit $H_2O_2$) zu OH umsetzt. Bevorzugte Bedingungen für die Metallierung sind die Umsetzung mit einem Lithiumalkyl wie n-BuLi, in THF, bei ca. -70°C, dann Zugabe des Elektrophils.

Das Verfahren und die anschließende Aufarbeitung des Reaktionsgemisches kann grundsätzlich als Batch-Reaktion oder in kontinuierlicher Reaktionsweise durchgeführt werden. Die kontinuierliche Reaktionsweise umfasst z. B. die Reaktion in einem kontinuierlichen Rührkesselreaktor, einer Rührkesselkaskade, einem Schlaufen- oder Querstromreaktor, einem Strömungsrohr oder in einem Mikroreaktor. Die Aufarbeitung der Reaktionsgemische erfolgt wahlweise, je nach Bedarf, durch Filtration über feste Phasen, Chromatographie, Separation zwischen unmischbaren Phasen (z. B. Extraktion), Adsorption an festen Trägern, Abdestillieren von Lösungsmitteln und/oder azeotropen Gemischen, selektive Destillation, Sublimation, Kristallisation, Cokristallisation oder durch Nanofiltration an Membranen.

Wie bereits erwähnt, können die Verbindungen der allgemeinen Formel I in flüssigkristallinen Medien verwendet werden. Gegenstand der vorliegenden Erfindung ist daher auch ein flüssigkristallines Medium mit mindestens zwei flüssigkristallinen Verbindungen, enthaltend mindestens eine Verbindung der allgemeinen Formel I.

Gegenstand der vorliegenden Erfindung sind auch flüssigkristalline Medien enthaltend neben einer oder mehreren erfindungsgemäßen Verbindungen der Formel I als weitere Bestandteile 2 bis 40, vorzugsweise 4 bis 30 Komponenten. Besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, 1,3-Dioxane, 2,5-Tetrahydropyrane, Phenyl- oder Cyclohexylbenzoate, Cyclohexancarbonsäurephenyl- oder-cyclohexylester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Biscyclohexylbenzole, 4',4'-Biscyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenylcyclohexyl)ethane, 1-Cyclohexyl-2-biphenylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch einfach oder mehrfach fluoriert sein.

Die wichtigsten als weitere Bestandteile erfindungsgemäßer Medien in Frage kommenden Verbindungen lassen sich durch die Formeln (II), (III), (IV), (V) und (VI) charakterisieren:

R'-L-E-R" (II)

R'-L-COO-E-R" (III)

R'-L-OOC-E-R" (IV)

R'-L-$CH_2CH_2$-E-R" (V)

R'-L-$CF_2O$-E-R" (VI)

In den Formeln (II), (III), (IV), (V) und (VI) bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -Thp-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl, Thp Tetrahydropyran-2,5-diyl und G 2-(trans-1,4-Cyclohexyl)-ethyl, Pyrimidin-2,5-diyl, Pyridin-2,5-diyl, 1,3-Dioxan-2,5-diyl oder Tetrahydropyran-2,5-diyl bedeuten.

Vorzugsweise ist einer der Reste L und E Cyc oder Phe. E ist vorzugsweise Cyc, Phe oder Phe-Cyc. Vorzugsweise enthalten die erfindungsgemäßen Medien eine oder mehrere Komponenten ausgewählt aus den Verbindungen der Formeln (II), (III), (IV), (V) und (VI), worin L und E ausgewählt sind aus der Gruppe Cyc und Phe und gleichzeitig eine oder mehrere Komponenten ausgewählt aus den Verbindungen der Formeln (II), (III), (IV), (V) und (VI), worin einer der Reste L und E ausgewählt ist aus der Gruppe Cyc und Phe und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln (II), (III), (IV), (V) und (VI), worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln (II), (III), (IV), (V) und (VI) jeweils unabhängig voneinander Alkyl, Alkenyl, Alkoxy, Alkoxyalkyl (Oxaalkyl), Alkenyloxy oder Alkanoyloxy mit bis zu 8 C-Atomen. Im folgenden wird diese kleinere Untergruppe Gruppe A genannt und die Verbindungen werden mit den Teilformeln (IIa), (IIIa), (IVa), (Va) und (VIa) bezeichnet. Bei den meisten dieser Verbindungen sind R' und R" voneinander verschieden, wobei einer dieser Reste meist Alkyl, Alkenyl, Alkoxy oder Alkoxyalkyl (Oxaalkyl) ist.

In einer anderen als Gruppe B bezeichneten kleineren Untergruppe der Verbindungen der Formeln (II), (III), (IV), (V) und (VI) bedeutet E

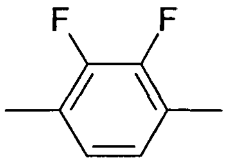

.

In den Verbindungen der Gruppe B, die mit den Teilformeln (IIb), (IIIb), (IVb), (Vb) und (VIb) bezeichnet werden, haben R' und R" die bei den Verbindungen der Teilformeln (IIa) bis (VIa) angegebene Bedeutung und sind vorzugsweise Alkyl, Alkenyl, Alkoxy oder Alkoxyalkyl (Oxaalkyl).

In einer weiteren kleineren Untergruppe der Verbindungen der Formeln (II), (III), (IV), (V) und (VI) bedeutet R" -CN. Diese Untergruppe wird im Folgenden als Gruppe C bezeichnet und die Verbindungen dieser Untergruppe werden entsprechend mit Teilformeln (IIc), (IIIc), (IVc), (Vc) und (VIc) beschrieben. In den Verbindungen der Teilformeln (IIc), (IIIc), (IVc), (Vc) und (VIc) hat R' die bei den Verbindungen der Teilformeln (IIa) bis (VIa) angegebene Bedeutung und ist vorzugsweise Alkyl, Alkenyl, Alkoxy oder Alkoxyalkyl (Oxaalkyl).

Neben den bevorzugten Verbindungen der Gruppen A, B und C sind auch andere Verbindungen der Formeln (II), (III), (IV), (V) und (VI) mit anderen Varianten der vorgesehenen Substituenten gebräuchlich. All diese Substanzen sind nach literaturbekannten Methoden oder in Analogie dazu erhältlich.

Die erfindungsgemäßen Medien enthalten neben den erfindungsgemäßen Verbindungen der allgemeinen Formel I vorzugsweise eine oder mehrere Verbindungen aus den Gruppen A, B und/oder C. Die Massenanteile der Verbindungen aus diesen Gruppen an den erfindungsgemäßen Medien betragen:

Gruppe A:

0 bis 90 %, vorzugsweise 20 bis 90 %, insbesondere 30 bis 90 %.

Gruppe B:

0 bis 80 %, vorzugsweise 10 bis 80 %, insbesondere 10 bis 70 %.

Gruppe C:

0 bis 80 %, vorzugsweise 5 bis 80 %, insbesondere 5 bis 50 %.

Die erfindungsgemäßen Medien enthalten vorzugsweise 1 bis 40 %, besonders bevorzugt 5 bis 30 % an den erfindungsgemäßen Verbindungen der Formel I. Die Medien enthalten vorzugsweise eine, zwei, drei, vier oder fünf erfindungsgemäße Verbindungen der Formel I.

Die Herstellung der erfindungsgemäßen Medien erfolgt in an sich üblicher Weise. In der Regel werden die Komponenten ineinander gelöst, vorzugsweise bei erhöhter Temperatur. Durch geeignete Zusätze können die flüssigkristallinen Phasen der vorliegenden Erfindung so modifiziert werden, dass sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die Verbindungen der Formel I eignen sich wegen ihres negativen $\Delta\varepsilon$ insbesondere für eine Verwendung in VA-TFT-Displays.

Gegenstand der vorliegenden Erfindung sind daher auch elektrooptische Anzeigeelemente, enthaltend ein erfindungsgemäßes flüssigkristallines Medium. Bevorzugt ist das Anzeigeelement ein VA-TFT-Anzeigeelement (VA: vertical alignment; TFT: thin film transistor).

Weitere Kombinationen der Ausführungsformen und Varianten der Erfindung gemäß der Beschreibung ergeben sich aus den Ansprüchen.

Weitere Ausführungsformen der vorliegenden Erfindung ergeben sich aus den Ansprüchen und aus Kombinationen zweier oder mehrerer dieser Ansprüche.

Die Erfindung wird im Folgenden anhand von Ausführungsbeispielen näher erläutert, ohne dadurch jedoch eingeschränkt werden zu sollen. Der Fachmann wird in der Lage sein, den Beispielen Details zur Durchführung zu entnehmen, die in der allgemeinen Beschreibung nicht im Einzelnen aufgeführt sind, sie nach allgemeinen Fachkenntnissen zu verallgemeinern und auf seine spezielle Problemstellung anzuwenden.

Neben den üblichen und wohlbekannten Abkürzungen werden folgende Abkürzungen verwendet:

K: Kristalline Phase; N: Nematische Phase; Sm: Smektische Phase;
I: Isotrope Phase. Die Zahlen zwischen diesen Symbolen geben die Übergangstemperaturen der betreffenden Substanz wieder. Temperaturangaben sind, soweit nichts anderes angegeben, in °C.

Die Bestimmung physikalischer, physikochemischer beziehungsweise elektrooptischer Parameter erfolgt nach allgemein bekannten Verfahren, wie sie unter anderem beschrieben sind in der Broschüre "Merck Liquid Crystals - Licristal® - Physical Properties of Liquid Crystals - Description of the Measurements Methods", 1998, Merck KGaA, Darmstadt.

Vor- und nachstehend bedeutet $\Delta n$ die optische Anisotropie (589 nm, 20 °C) und $\Delta\varepsilon$ die dielektrische Anisotropie (1 kHz, 20 °C). Die dielektrische Anisotropie $\Delta\varepsilon$ wird bei 20°C und 1 kHz bestimmt. Die optische Anisotropie $\Delta n$ wird bei 20°C und einer Wellenlänge von 589,3 nm bestimmt.

Die $\Delta\varepsilon$- und $\Delta n$-Werte, der extrapolierte Klärpunkt (Klp.) sowie die Rotationsviskosität ($\gamma_1$) der erfindungsgemäßen Verbindungen werden durch lineare Extrapolation aus flüssigkristallinen Mischungen erhalten, die zu 5 bis 10 % aus der jeweiligen erfindungsgemäßen Verbindung und zu 90-95 % aus der kommerziell erhältlichen Flüssigkristallmischung ZLI-2857 (für $\Delta\varepsilon$, Klp.) bzw. ZLI-4792 (für $\Delta n$, $\gamma_1$) bestehen (Mischungen Fa. Merck KGaA, Darmstadt).

Nachstehend bedeuten die Abkürzungen:

| | |
|---|---|
| MTB | Methyl-tert-butylether |

| | |
|---|---|
| THF | Tetrahydrofuran |
| DMF | Dimethylformamid |
| DMAP | 4-(Dimethylamino)pyridin |
| ges. Lsg. | gesättigte Lösung |
| *n*-BuLi, BuLi | *n*-Butyllithium, Lösung in Hexan |
| RT | Raumtemperatur, ca. 20 °C |
| Schmp. | Schmelzpunkt |

**Beispiele**

Die Ausgangssubstanzen können nach allgemein zugänglichen Literaturvorschriften oder käuflich erhalten werden.

**Beispiel 1:** 3-Ethoxy-4,6-difluor-7-pentyl-dibenzothiophen

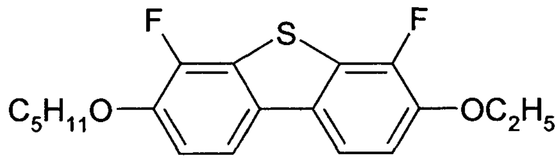

Schritt 1

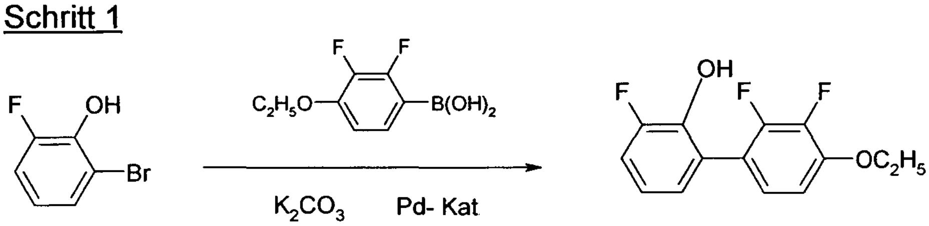


0,058 mol 2-Brom-6-fluorphenol wird in 100ml THF gelöst und mit 40 ml Wasser und 0,09 mol Kaliumcarbonat versetzt. Nach Erwärmen auf Siedetemperatur setzt man 0,3mmol Tris(dibenzylidenaceton)dipalladium(0) sowie 0,9 mmol cataCXium® A (Di(1-adamantyl)-n-butylphosphin) zu, und tropft innerhalb einer halben Stunde eine Lösung von 0,062 mol (4-Ethoxy-2,3-difluoro-phenyl)-dimethoxy-boran, gelöst in 100 ml THF, zu. Man kocht noch 16 Stunden am Rückfluss, versetzt dann mit Wasser und MTB und arbeitet extraktiv auf. Das Rohprodukt 4'-Ethoxy-3,2',3'-trifluoro-biphenyl-2-ol wird chromatographisch gereinigt (Lauf-mittel: Chlorbutan).
Man erhält weiße Kristalle.

Schritt 2

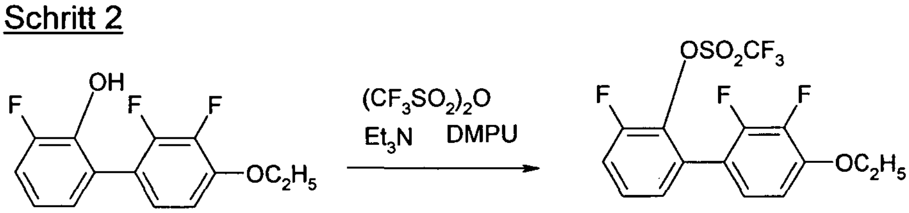


0,022 mol 4'-Ethoxy-3,2',3'-trifluoro-biphenyl-2-ol, 0,036 mol Triethylamin und 0,6 mmol DMAP werden in 50ml Dichlormethan gelöst. Zu dieser Lösung tropft man bei 5-10°C, innerhalb einer halben Stunde, 0,03 mol Trifluormethansulfonsäureanhydrid. Man rührt noch eine Stunde ohne weitere Kühlung und filtriert den Ansatz mit Dichlormethan über eine Säule mit Kieselgel. Nach Eindampfen des Filtrates erhält man das Trifluormethylsulfonat des 4'-Ethoxy-3,2',3'-trifluoro-biphenyl-2-ol.

Schritt 3

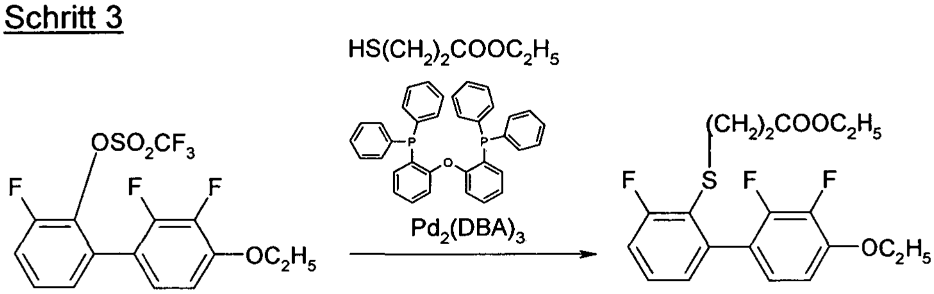


0,022 mol Trifluormethansulfonat des 4'-Ethoxy-3,2',3'-trifluoro-biphenyl-2-ol, und 0,024 mol Ethylmercaptopropionat werden in 50 ml trockenem Toluol gelöst und mit 2,2 mmol Bis(2-diphenylphosphinophenyl)ether, 1,1 mmol Tris-(dibenzylidenaceton)dipalladium(0) und 0,055 mol Kaliumcarbonat 24 Stunden am Rückfluss gekocht. Man versetzt das Reaktionsgemisch mit Wasser und MTB und arbeitet extraktiv auf. Die organische Phase wird eingeengt und das Produkt durch Säulenchromatographie mit 1-Chlorbutan über Kieselgel isoliert. Man erhält 0,014 m 3-(4'-Ethoxy-3,2',3'-trifluoro-biphenyl-2-ylsulfanyl)-propionsäureethylester.

Schritt 4

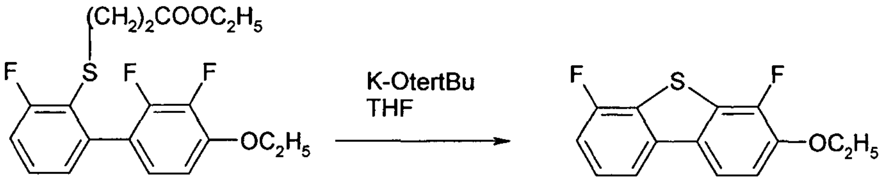


0,014 mol 3-(4'-Ethoxy-3,2',3'-trifluoro-biphenyl-2-ylsulfanyl)-propionsäureethyl-ester werden mit 0,017 mol Kalium-tert.-butylat in 50 ml THF 14 Stunden am Rückfluss gekocht. Man versetzt das Reaktionsgemisch mit Wasser und MTB und arbeitet extraktiv auf. Die organische Phase wird eingeengt und das Produkt durch Säulenchromatographie mit 1-Chlorbutan über Kieselgel isoliert. Man erhält 3-Ethoxy-4,6-difluor-dibenzothiophen.

Schritt 5

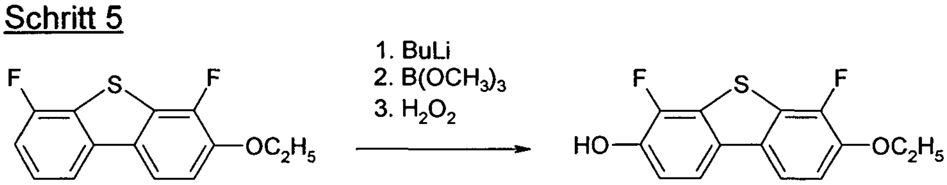


0,013 mol 3-Ethoxy-4,6-difluor-dibenzothiophen werden in 50 ml THF gelöst und bei -70°C mit 11 ml (0,017 mol) n-BuLi (1,6 M in Hexan) tropfenweise versetzt. Man rührt noch 30 Minuten bei -70°C nach und setzt dann bei gleicher Temperatur 0,017, mol Trimethylborat (gelöst in etwas THF) zu. Man erwärmt das Reaktionsgemisch auf Raumtemperatur und versetzt dann mit einem Gemisch aus 2 ml Essigsäure und 2,5 ml Wasser. Danach tropft man 3 ml 30%iges Wasserstoffperoxid bei max. 45°C zu. Nach weiteren 14 Stunden Nachrührzeit versetzt man das Reaktionsgemisch mit Wasser und MTB und arbeitet extraktiv auf. Die organische Phase wird eingeengt und das Produkt durch Säulenchromatographie mit Dichlormethan über Kieselgel isoliert. Man erhält 0,011 mol 7-Ethoxy-4,6-difluor-dibenzothiophen-3-ol.

Schritt 6

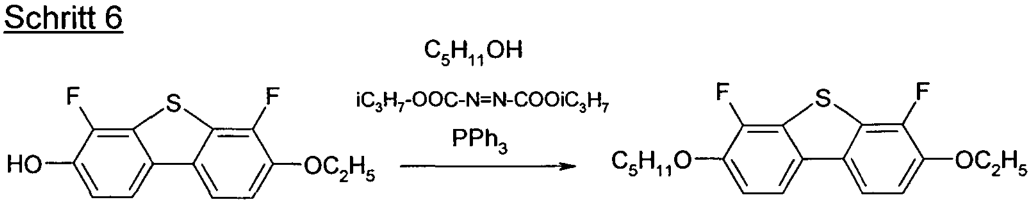


0,011 mol 3-Ethoxy-4,6-difluor-dibenzothiophen-3-ol, 0,018 mol Pentan-1-ol und 0,017 mol Triphenylphosphin werden in 50 ml THF gelöst und bei Raumtemperatur mit 0,017 mol Diisopropylazodicarboxylat tropfenweise versetzt.

Man rührt noch 14 Stunden nach und versetzt danach das Reaktionsgemisch mit Wasser und MTB und arbeitet extraktiv auf. Die organische Phase wird eingeengt und das Produkt durch Säulenchromatographie mit 1-Chlorbutan über Kieselgel isoliert. Man erhält 3,6 g (0,01 mol) 3-Ethoxy-4,6-difluor-7-pentyloxydibenzothiophen. Schmelzpunkt: 77°C.

Phasen: K 77 I (vgl. auch Tabelle).

Analog zu Beispiel 1 werden die folgenden Verbindungen hergestellt:

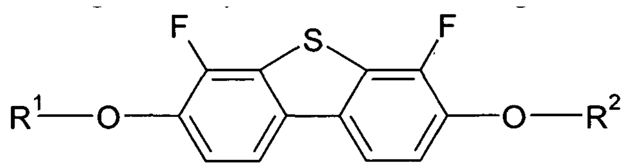

Die Reste $R^{1/2}$ sind geradkettig, d.h. unverzweigt, soweit nicht anders angegeben. Die Stoffdaten ergeben sich aus Tabelle 1.

Tabelle 1.

| $R^1$ | $R^2$ | Schmp. [°C] | $\Delta\varepsilon$ | $\Delta n$ | $\gamma_1$ [mPa·s] | Klp. [°C] |
|---|---|---|---|---|---|---|
| $-CH_3$ | $-CH_3$ | | | | | |
| $-CH_3$ | $-C_2H_5$ | | | | | |
| $-CH_3$ | $-C_3H_7$ | | | | | |
| $-CH_3$ | $-C_4H_9$ | | | | | |
| $-CH_3$ | $-C_5H_{11}$ | | | | | |
| $-CH_3$ | $-C_6H_{13}$ | | | | | |
| $-C_2H_5$ | $-C_2H_5$ | 126 | | | | |
| $-C_2H_5$ | $-C_3H_7$ | 100 | -14 | 0,214 | 168 | 136 |
| $-C_2H_5$ | $-C_4H_9$ | 79 | -14 | 0,213 | 164 | 127 |
| | $-CH_2CH(CH_3)_2$ | | | | | |
| $-C_2H_5$ | $-(CH_2)_2CH=CH_2$ | | | | | |
| $-C_2H_5$ | $-(CH_2)_2CH(CH_3)_2$ | | | | | |
| $-C_2H_5$ | $-C_5H_{11}$ | 77 | -13 | 0,196 | 197 | 120 |
| $-C_2H_5$ | $-C_6H_{13}$ | 77 | -13 | 0,197 | 183 | 121 |
| $-C_2H_5$ | $-C_7H_{15}$ | 86 | -12 | 0,187 | 222 | 114 |
| $-C_2H_5$ | $-(CH_2)_3CH(CH_3)_2$ | | | | | |
| $-C_3H_7$ | $-C_3H_7$ | 97 | -14 | 0,206 | 189 | 120 |
| $-C_3H_7$ | $-C_4H_9$ | 85 | -13 | 0,199 | 186 | 118 |
| $-C_3H_7$ | $-C_5H_{11}$ | 80 | -13 | 0,189 | 179 | 111 |
| $-C_3H_7$ | $-C_6H_{13}$ | 80 | -13 | 0,191 | 210 | 113 |
| $-C_3H_7$ | $-C_7H_{15}$ | 77 | -12 | 0,181 | 226 | 108 |
| $-C_4H_9$ | $-C_4H_9$ | 85 | -13 | 0,194 | 195 | 115 |
| $-C_4H_9$ | $-C_5H_{11}$ | 84 | -12 | 0,186 | 176 | 112 |
| $-C_4H_9$ | $-(CH_2)_2CH=CHCH_3$ *) | | | | | |
| $-C_4H_9$ | $-C_6H_{13}$ | 74 | -12 | 0,182 | 202 | 112 |
| $-C_4H_9$ | $-C_7H_{15}$ | 68 | -12 | 0,177 | 204 | 109 |

(fortgesetzt)

| $R^1$ | $R^2$ | Schmp. [°C] | Δε | Δn | $\gamma_1$ [mPa·s] | Klp. [°C] |
|---|---|---|---|---|---|---|
| $-C_5H_{11}$ | $-C_5H_{11}$ | 85 | -14 | 0,163 | 142 | 100 |
| $-C_5H_{11}$ | $-C_6H_{13}$ | | | | | |
| $-C_6H_{13}$ | $-C_6H_{13}$ | 85 | -12 | 0,166 | 183 | 106 |
| *) trans-Isomer | | | | | | |

Analog zu Beispiel 1 und Schema 2 werden die folgenden Verbindungen hergestellt:

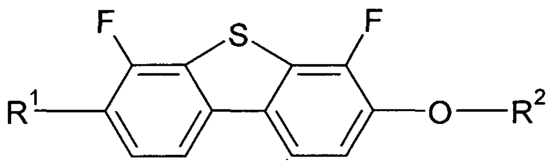

Die Reste $R^{1/2}$ sind geradkettig, d.h. unverzweigt, soweit nicht anders angegeben. Die Stoffdaten ergeben sich aus Tabelle 2.

Tabelle 2.

| $R^1$ | $R^2$ | Schmp. [°C] | Δε | Δn | $\gamma_1$ [mPa·s] | Klp. [°C] |
|---|---|---|---|---|---|---|
| $-CH_3$ | $-CH_3$ | | | | | |
| $-CH_3$ | $-C_2H_5$ | 105 | -9,8 | 0,219 | 130 | 100 |
| $-CH_3$ | $-C_3H_7$ | 105 | -9,8 | 0,205 | 111 | 97 |
| $-CH_3$ | $-C_4H_9$ | 87 | -8,9 | 0,195 | 107 | 101 |
| $-CH_3$ | $-C_5H_{11}$ | 73 | -9,0 | 0,195 | 110 | 81 |
| $-CH_3$ | $-C_6H_{13}$ | | | | | |
| $-C_2H_5$ | $-CH_3$ | | | | | |
| $-C_2H_5$ | $-C_2H_5$ | 101 | -9,2 | 0,203 | 123 | 75 |
| $-C_2H_5$ | $-C_3H_7$ | 70 | -8,6 | 0,188 | 118 | 68 |
| $-C_2H_5$ | $-C_4H_9$ | 69 | -8,7 | 0,187 | 116 | 69 |
| $-C_2H_5$ | $-CH_2CH(CH_3)_2$ | | | | | |
| $-C_2H_5$ | $-(CH_2)_2CH{=}CH_2$ | | | | | |
| $-C_2H_5$ | $-(CH_2)_2CH(CH_3)_2$ | | | | | |
| $-C_2H_5$ | $-C_5H_{11}$ | | | | | |
| $-C_2H_5$ | $-C_6H_{13}$ | | | | | |
| $-C_2H_5$ | $-(CH_2)_3CH(CH_3)_2$ | | | | | |
| $-C_3H_7$ | $-CH_3$ | | | | | |
| $-C_3H_7$ | $-C_2H_5$ | 105 | -9,6 | 0,201 | 147 | 78 |
| $-C_3H_7$ | $-C_3H_7$ | 73 | -8,7 | 0,188 | 139 | 74 |
| $-C_3H_7$ | $-C_4H_9$ | 72 | -8,7 | 0,184 | 133 | 75 |
| $-C_3H_7$ | $-(CH_2)_2CH{=}CH_2$ | | | | | |
| $-C_3H_7$ | $-C_5H_{11}$ | | | | | |
| $-C_3H_7$ | $-C_6H_{13}$ | | | | | |

(fortgesetzt)

| $R^1$ | $R^2$ | Schmp. [°C] | Δε | Δn | $\gamma_1$ [mPa·s] | Klp. [°C] |
|---|---|---|---|---|---|---|
| $-C_4H_9$ | $-CH_3$ | | | | | |
| $-C_4H_9$ | $-C_2H_5$ | 80 | -9,2 | 0,191 | 137 | 73 |
| $-C_4H_9$ | $-C_3H_7$ | 62 | -8,9 | 0,180 | 135 | 67 |
| $-(CH_2)_2-CH=CH_2$ | $-C_2H_5$ | | | | | |
| $-C_4H_9$ | $-C_4H_9$ | 62 | -8,6 | 0,177 | 123 | 64 |
| $-C_4H_9$ | $-C_5H_{11}$ | | | | | |
| $-C_4H_9$ | $-(CH_2)_2CH=CHCH_3$ *) | | | | | |
| $-C_4H_9$ | $-C_6H_{13}$ | | | | | |
| $-C_5H_{11}$ | $-CH_3$ | | | | | |
| $-C_5H_{11}$ | $-C_2H_5$ | 71 | -8,9 | 0,191 | 158 | 80 |
| $-(CH_2)_2-CH=CH-CH_3$ *) | $-C_2H_5$ | | | | | |
| $-C_5H_{11}$ | $-C_3H_7$ | 71 | -7,7 | 0,181 | 143 | 72 |
| $-C_5H_{11}$ | $-C_4H_9$ | 69 | -8,6 | 0,179 | 144 | 73 |
| $-C_5H_{11}$ | $-(CH_2)_2CH=CH_2$ | | | | | |
| $-C_5H_{11}$ | $-C_6H_{13}$ | | | | | |
| *) trans-Isomer | | | | | | |

Analog zu Beispiel 1 und Schema 3 werden die folgenden Verbindungen hergestellt:

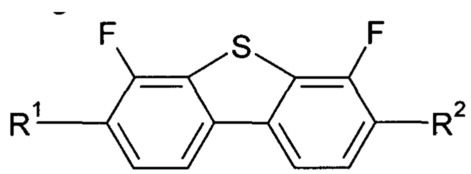

Die Reste $R^{1/2}$ sind geradkettig, d.h. unverzweigt, soweit nicht anders angegeben. Die Stoffdaten ergeben ssich aus Tabelle 3.

Tabelle 3.

| $R^1$ | $R^2$ | Schmp. [°C] | Δε | Δn | $\gamma_1$ [mPa·s] | Klp. [°C] |
|---|---|---|---|---|---|---|
| $-CH_3$ | $-CH_3$ | | | | | |
| $-CH_3$ | $-C_2H_5$ | | | | | |
| $-CH_3$ | $-C_3H_7$ | | | | | |
| $-CH_3$ | $-C_4H_9$ | | | | | |
| $-CH_3$ | $-C_5H_{11}$ | 50 | -5,6 | 0,170 | 86 | 42 |
| $-CH_3$ | $-C_6H_{13}$ | | | | | |
| $-CH_3$ | $-(CH_2)_2CH=CH_2$ | 42 | -6,2 | 0,185 | 76 | 48 |
| $-CH_3$ | $-(CH_2)_2CH=CHCH_3$ *) | | | | | |
| $-C_2H_5$ | $-C_2H_5$ | | | | | |
| $-C_2H_5$ | $-C_3H_7$ | 66 | -7,2 | 0,166 | 71 | 15 |
| $-C_2H_5$ | $-C_4H_9$ | | | | | |

(fortgesetzt)

| $R^1$ | $R^2$ | Schmp. [°C] | Δε | Δn | $\gamma_1$ [mPa·s] | Klp. [°C] |
|---|---|---|---|---|---|---|
| $-C_2H_5$ | $-CH_2CH(CH_3)_2$ | | | | | |
| $-C_2H_5$ | $-(CH_2)_2CH=CH_2$ | | | | | |
| $-C_2H_5$ | $-(CH_2)_2CH(CH_3)_2$ | | | | | |
| $-C_2H_5$ | $-C_5H_{11}$ | | | | | |
| $-C_2H_5$ | $-C_6H_{13}$ | | | | | |
| $-C_2H_5$ | $-(CH_2)_3CH(CH_3)_2$ | | | | | |
| $-C_3H_7$ | $-C_3H_7$ | | | | | |
| $-C_3H_7$ | $-C_4H_9$ | 57 | -4,9 | 0,154 | 80 | 14 |
| $-C_3H_7$ | $-(CH_2)_2CH=CH_2$ | | | | | |
| $-C_3H_7$ | $-C_5H_{11}$ | | | | | |
| $-C_3H_7$ | $-C_6H_{13}$ | | | | | |
| $-C_4H_9$ | $-C_4H_9$ | | | | | |
| $-(CH_2)_2-CH=CH_2$ | $-(CH_2)_2CH=CH_2$ | 75 | -6,6 | 0,184 | 61 | 32 |
| $-C_4H_9$ | $-C_5H_{11}$ | | | | | |
| $-C_4H_9$ | $-(CH_2)_2CH=CHCH_3$*) | | | | | |
| $-C_4H_9$ | $-C_6H_{13}$ | | | | | |
| $-C_5H_{11}$ | $-(CH_2)_2CH=CH_2$ | | | | | |
| $-C_5H_{11}$ | $-C_6H_{13}$ | | | | | |
| *) trans-Isomer | | | | | | |

Analog zu Beispiel 1 werden die folgenden Verbindungen hergestellt:

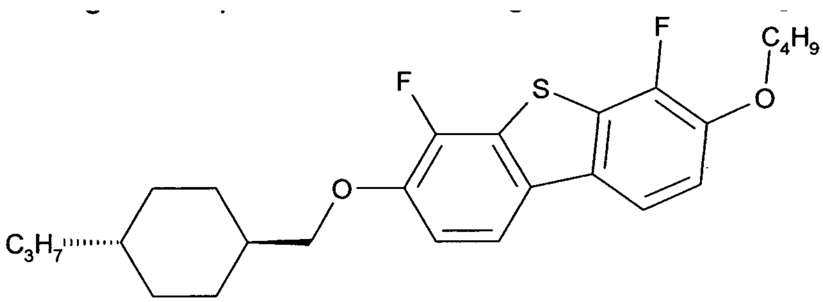

Phasen: K 111 N 170 I (Schmp. 111°C)
Δε: -12
Δn: 0,191
$\gamma_1$ [mPa·s]: 954

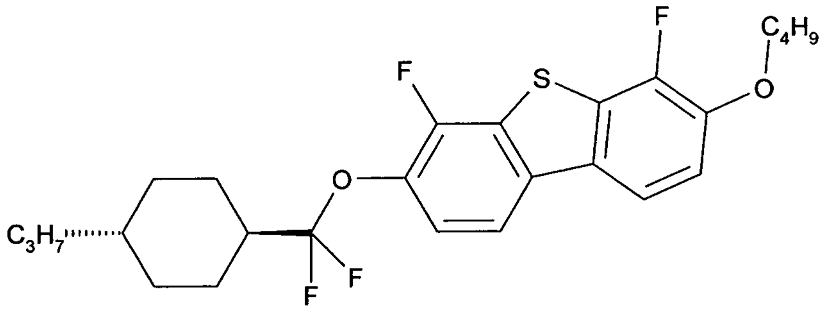

Phasen: K 81 N 175 I (Schmp. 81 °C)
Δε: -6,4
Δn: 0,163
$\gamma_1$ [mPa·s]: 715

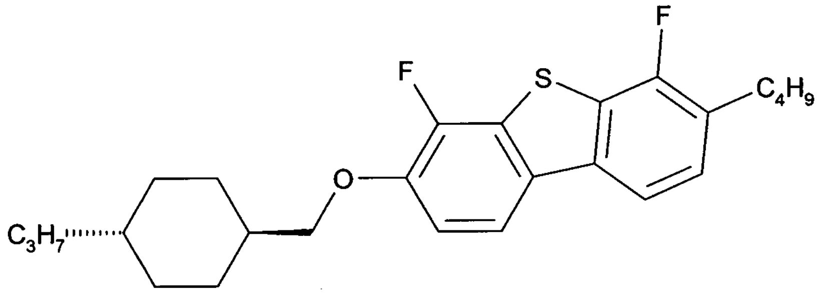

Phasen: K 85 N 135 I (Schmp. 85°C)
Δε: -9,0
Δn: 0,173
$\gamma_1$ [mPa·s]: 769

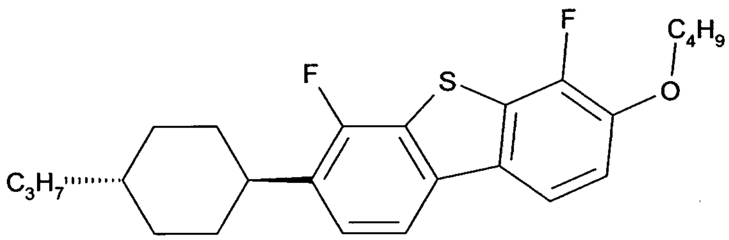

Phasen: K 99 N 212 I (Schmp. 99°C)
Δε: -9,4
Δn: 0,201
$\gamma_1$ [mPa·s]: 1019

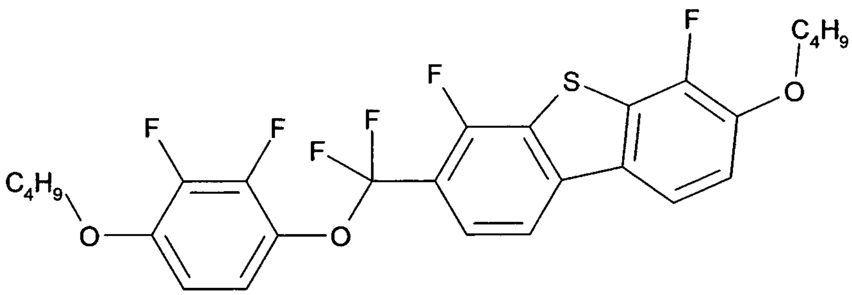

Phasen: K 114 N 133 I (Schmp. 114°C)

Δε: -10
Δn: 0,177
$\gamma_1$ [mPa·s]: 869

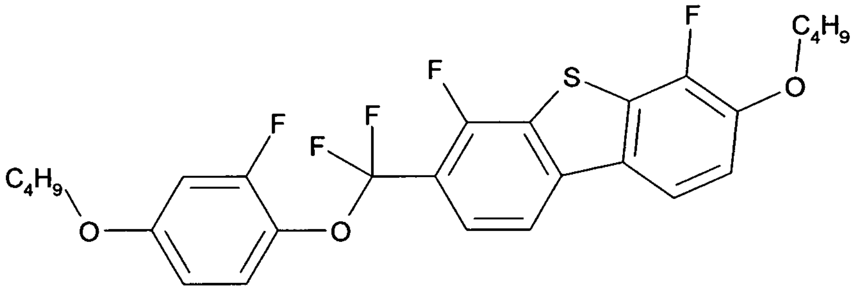

Phasen: K 74 N 132 I (Schmp. 74°C)
Δε: -7,2
Δn: 0,190
$\gamma_1$ [mPa·s]: 1086

**Patentansprüche**

**1.** Verbindungen der Formel I:

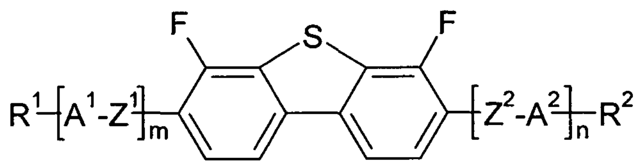

I

worin

m und n jeweils unabhängig voneinander 0, 1 oder 2 sind,
$R^1$ und $R^2$ unabhängig voneinander einen Alkyl- oder Alkoxyrest mit 1 bis 15 C-Atomen, wobei in diesen Resten auch eine oder mehrere $CH_2$-Gruppen jeweils unabhängig voneinander durch -C=C-, $-CF_2O-$, $-OCF_2-$, -CH=CH-,

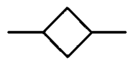 , 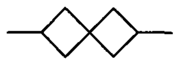 ,

-O-, -CO-O-, oder -O-CO- so ersetzt sein können, dass O-Atome nicht direkt miteinander verknüpft sind, und worin auch ein oder mehrere H-Atome durch Halogen ersetzt sein können,
$A^1$, und $A^2$ unabhängig voneinander einen Rest ausgewählt aus folgenden Gruppen

a) 1,4-Phenylen worin auch eine oder zwei CH-Gruppen durch N ersetzt sein können und worin auch ein oder mehrere H-Atome durch eine Gruppe L ersetzt sein können,
b) der Gruppe bestehend aus trans-1,4-Cyclohexylen und 1,4-Cyclohexenylen , worin auch eine oder mehrere nicht benachbarte $CH_2$-Gruppen durch -O- und/oder -S- ersetzt sein können und worin auch ein oder mehrere H-Atome durch F oder Cl ersetzt sein können, und
c) der Gruppe bestehend aus Tetrahydropyran-2,5-diyl, 1,3-Dioxan-2,5-diyl, Tetrahydrofuran-2,5-diyl, Cyclobutan-1,3-diyl, Piperidin-1,4-diyl, Thiophen-2,5-diyl und Selenophen-2,5-diyl, welche auch ein oder mehrfach durch ein Gruppe L substituiert sein können,

L bei jedem Auftreten unabhängig F, Cl, CN, SCN, $SF_5$ oder geradkettiges oder verzweigtes, jeweils optional fluoriertes, Alkyl, Alkoxy, Alkylcarbonyl, Alkoxycarbonyl, Alkylcarbonyloxy oder Alkoxycarbonyloxy mit 1 bis 12 C-Atomen, und

$Z^1$ und $Z^2$ unabhängig voneinander eine Einfachbindung, $-CF_2O-$, $-OCF_2-$, $-CH_2O-$, $-OCH_2-$, -(CO)O-, -O(CO)-, $-(CH_2)_4-$, $-CH_2CH_2-$, $-CF_2-CF_2-$, $-CF_2-CH_2-$, $-CH_2-CF_2-$, -CH=CH-, -CF=CF-, -CF=CH-, -CH=CF-, $-(CH_2)_3O-$, $-O(CH_2)_3-$, -C≡C-, -O-, $-CH_2-$, $-(CH_2)_3-$ oder $-CF_2-$, bevorzugt eine Einfachbindung,

bedeuten.

2. Verbindungen nach Anspruch 1, **dadurch gekennzeichnet, dass** in der Formel I die Summe m + n den Wert 0 oder 1 hat.

3. Verbindungen nach Anspruch 1 oder 2, **dadurch gekennzeichnet, dass**

$R^1$ und $R^2$ unabhängig voneinander einen unsubstituierten Alkylrest oder Alkoxyrest mit 1 bis 15 Kohlenstoffatomen oder einen Alkenyl-, Alkenyloxy- oder Alkinylrest mit 2 bis 15 C-Atomen, welche jeweils optional ein- oder mehrfach halogeniert sind,

bedeuten.

4. Verbindungen nach einem oder mehreren der Ansprüche 1 bis 3, **dadurch gekennzeichnet dass** in der Formel I m + n = 0 ist.

5. Verbindungen nach einem oder mehreren der Ansprüche 1 bis 4, **dadurch gekennzeichnet, dass** die Verbindungen der Formel I ausgewählt sind aus den Unterformeln IA bis IC

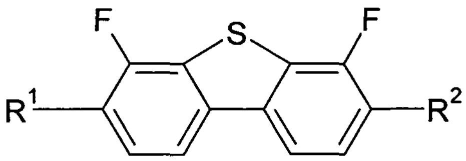

IA

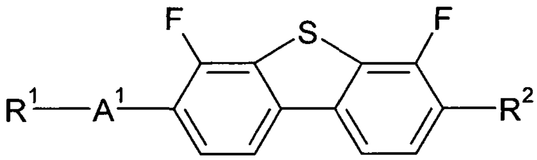

IB

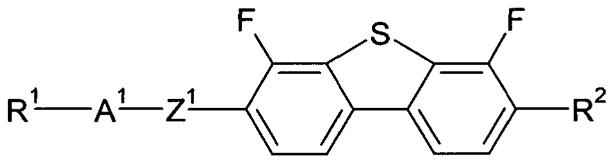

IC

worin

$R^1$, $R^2$, $A^1$ und $Z^1$ die Bedeutungen wie für die Formel I nach Anspruch 1 besitzen.

6. Verbindungen nach einem oder mehreren der Ansprüche 1 bis 5, **dadurch gekennzeichnet, dass** die Verbindungen ausgewählt sind aus Verbindungen der Formeln IA-1 bis IA-10,

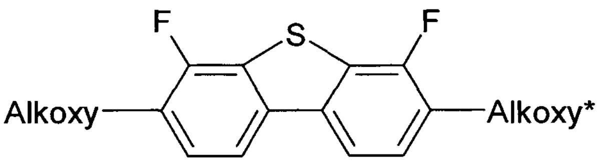

IA-1

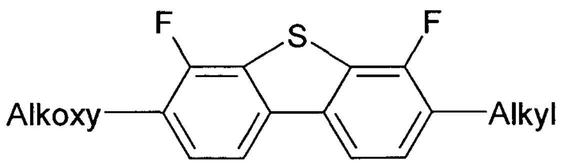

IA-2

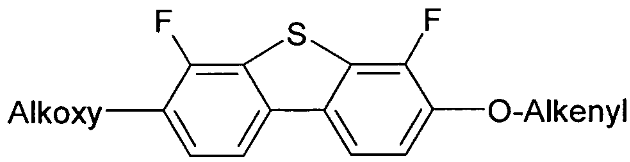

IA-3

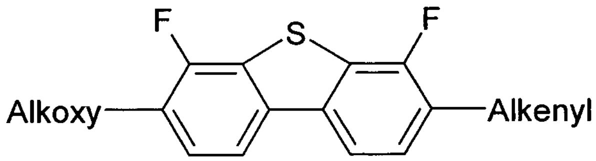

IA-4

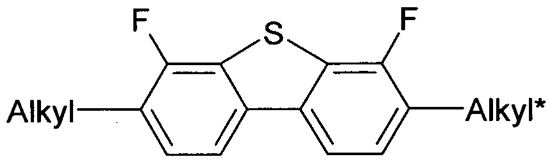

IA-5

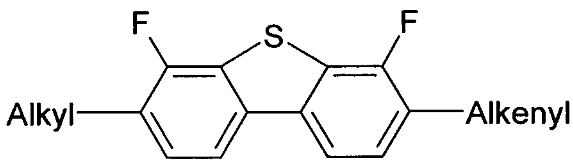

IA-6

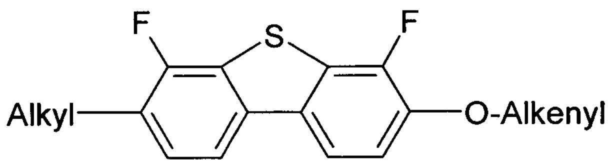

IA-7

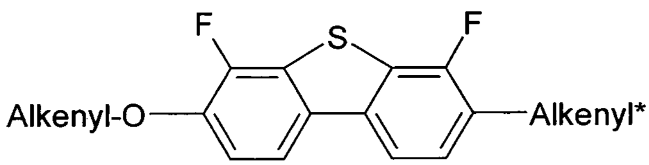

IA-8

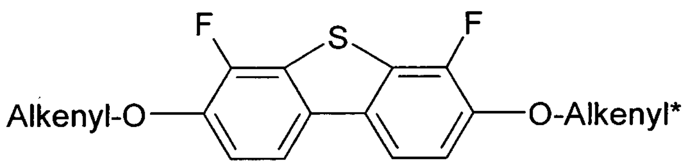

IA-9

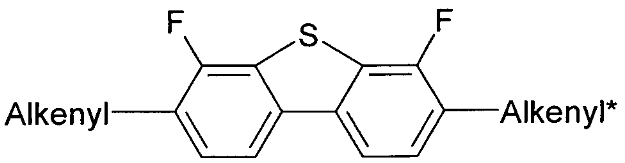

IA-10,

worin Alkyl und Alkyl* jeweils unabhängig voneinander einen geradkettigen Alkylrest mit 1-7 C-Atomen, Alkenyl und Alkenyl* jeweils unabhängig voneinander einen geradkettigen Alkenylrest mit 2-7 C-Atomen, Alkoxy und Alkoxy* jeweils unabhängig voneinander einen geradkettigen Alkoxyrest mit 1-7 C-Atomen bedeuten.

**7.** Verbindungen nach einem oder mehreren der Ansprüche 1 bis 6, **dadurch gekennzeichnet, dass**

$R^1$ und $R^2$ unabhängig voneinander einen Alkoxy- oder Alkylrest mit 1 bis 7 oder einen Alkenylrest mit 2 bis 7 Kohlenstoffatomen bedeuten.

**8.** Verbindungen nach einem oder mehreren der Ansprüche 1 bis 7, **dadurch gekennzeichnet, dass** $R^1$ und $R^2$ jeweils eine Alkoxygruppe mit 1 bis 7 Kohlenstoffatomen bedeuten.

**9.** Verbindungen nach einem oder mehreren der Ansprüche 1 bis 7, **dadurch gekennzeichnet, dass**

$$m + n = 1 \text{ oder } 2 \text{ ist, und}$$

oder 2 ist, und
der Ring $A^1$ jeweils unabhängig ausgewählt ist aus den Teilstrukturen

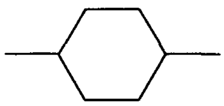 , 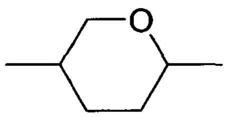 , 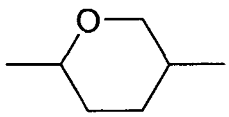 ,

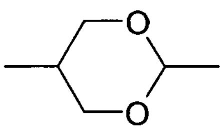 , 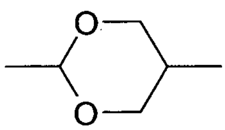 , 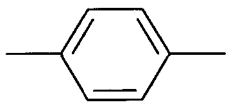 ,

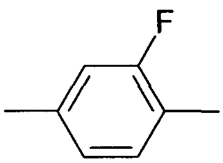 , 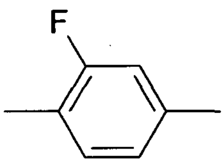 oder 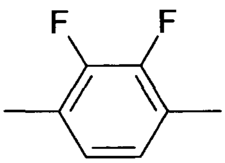 .

**10.** Verwendung einer oder mehrerer Verbindungen nach einem oder mehreren der Ansprüche 1 bis 9 in flüssigkristallinen Medien.

**11.** Flüssigkristallines Medium enthaltend mindestens zwei Verbindungen, **dadurch gekennzeichnet, dass** es mindestens eine Verbindung nach einem oder mehreren der Ansprüche 1 bis 9 enthält.

**12.** Elektrooptisches Anzeigeelement, enthaltend ein flüssigkristallines Medium nach Anspruch 11.

**13.** Verfahren zur Herstellung von Verbindungen der Formel I nach einem oder mehreren der Ansprüche 1 bis 9, **dadurch gekennzeichnet, dass** es einen Verfahrensschritt umfasst, wobei eine Verbindung der Formel (B)

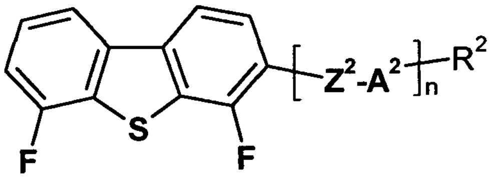

**(B)**

worin $Z^2$, $A^2$, n und $R^2$ unabhängig wie in Formel I nach einem der Ansprüche 1 bis 9 definiert sind, an der Position 3 mittels eines Deprotonierungsreagenzes deprotoniert und zu einer Verbindung der Formel (C)

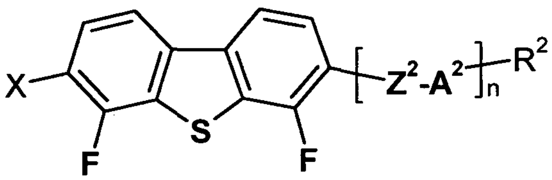

**(C)**

worin unabhängig X $B(OR)_2$, $-C(OH)R_2$, -(CO)OH, -(CO)Cl, OH oder

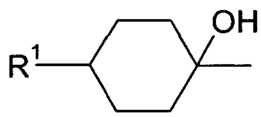

bedeutet,
R unabhängig H oder einen Alkylrest mit 1 bis 14 C-Atomen bedeutet, und
$Z^2$, $A^2$, n, $R^1$ und $R^2$ wie in Formel I definiert sind, umgesetzt wird, und in weiteren Verfahrensschritten zu einer Verbindung der Formel I umgesetzt wird.

**Claims**

**1.** Compounds of the formula I:

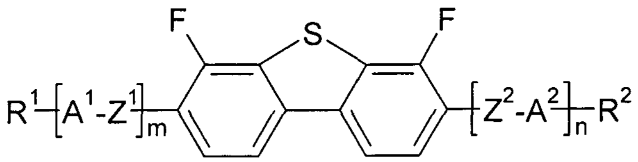

I

in which

m and n are each, independently of one another, 0, 1 or 2,
$R^1$ and $R^2$, independently of one another, denote an alkyl or alkoxy radical having 1 to 15 C atoms, where, in addition, one or more $CH_2$ groups in these radicals may each be replaced, independently of one another, by $-C\equiv C-$, $-CF_2O-$, $-OCF_2-$, -CH=CH-,

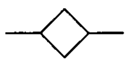 , 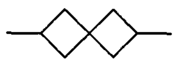 ,

-O-, -CO-O- or -O-CO- in such a way that O atoms are not linked directly to one another, and in which, in addition, one or more H atoms may be replaced by halogen,
$A^1$ and $A^2$, independently of one another, denote a radical selected from the following groups:

a) 1,4-phenylene, in which, in addition, one or two CH groups may be replaced by N and in which, in addition, one or more H atoms may be replaced by a group L,

b) the group consisting of trans-1,4-cyclohexylene and 1,4-cyclohexenylene, in which, in addition, one or more non-adjacent $CH_2$ groups may be replaced by -O- and/or -S- and in which, in addition, one or more H atoms may be replaced by F or Cl, and

c) the group consisting of tetrahydropyran-2,5-diyl, 1,3-dioxane-2,5-diyl, tetrahydrofuran-2,5-diyl, cyclobutane-1,3-diyl, piperidine-1,4-diyl, thiophene-2,5-diyl and selenophene-2,5-diyl, each of which may also be mono- or polysubstituted by a group L,

L on each occurrence, independently, denotes F, Cl, CN, SCN, $SF_5$ or straight-chain or branched, in each case optionally fluorinated alkyl, alkoxy, alkylcarbonyl, alkoxycarbonyl, alkylcarbonyloxy or alkoxycarbonyloxy having 1 to 12 C atoms, and

$Z^1$ and $Z^2$, independently of one another, denote a single bond, $-CF_2O-$, $-OCF_2-$, $-CH_2O-$, $-OCH_2-$, -(CO)O-, -O(CO)-, $-(CH_2)_4-$, $-CH_2CH_2-$, $-CF_2-CF_2-$, $-CF_2-CH_2-$, $-CH_2-CF_2-$, -CH=CH-, -CF=CF-, -CF=CH-, -CH=CF-, $-(CH_2)_3O-$, $-O(CH_2)_3-$, -C≡C-, -O-, $-CH_2-$, $-(CH_2)_3-$ or $-CF_2-$, preferably a single bond.

**2.** Compounds according to Claim 1, **characterised in that** the sum m + n in the formula I has the value 0 or 1.

**3.** Compounds according to Claim 1 or 2, **characterised in that**

$R^1$ and $R^2$, independently of one another, denote an unsubstituted alkyl radical or alkoxy radical having 1 to 15 carbon atoms or an alkenyl, alkenyloxy or alkynyl radical having 2 to 15 C atoms, each of which is optionally mono- or polyhalogenated.

**4.** Compounds according to one or more of Claims 1 to 3, **characterised in that**, in the formula I, m + n = 0.

**5.** Compounds according to one or more of Claims 1 to 4, **characterised in that** the compounds of the formula I are selected from the sub-formulae IA to IC

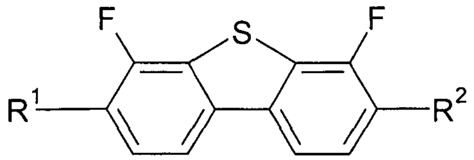

IA

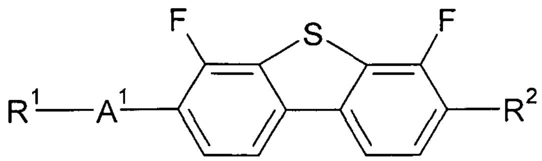

IB

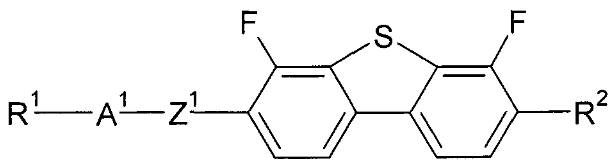

IC

in which

$R^1$, $R^2$, $A^1$ and $Z^1$ have the meanings as for the formula I according to Claim 1.

**6.** Compounds according to one or more of Claims 1 to 5, **characterised in that** the compounds are selected from compounds of the formulae IA-1 to IA-10,

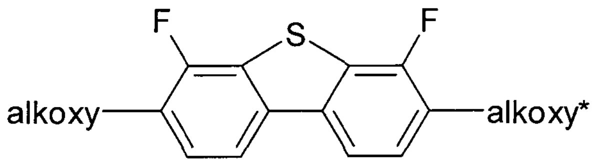

IA-1

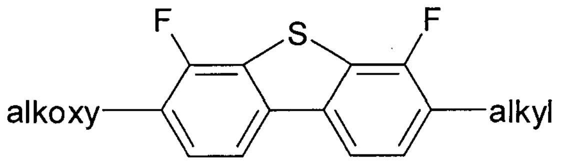

IA-2

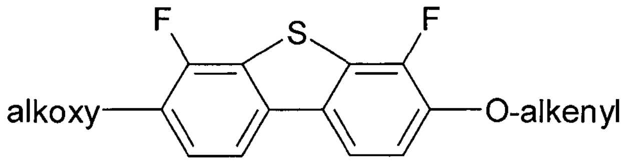

IA-3

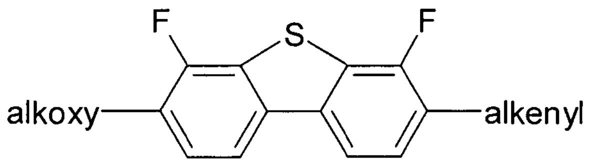

IA-4

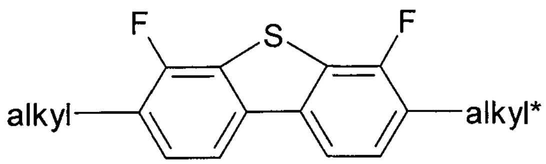

IA-5

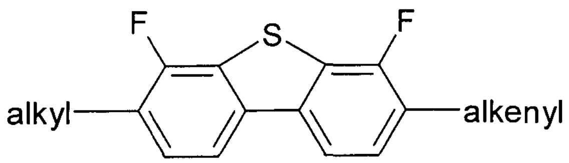

IA-6

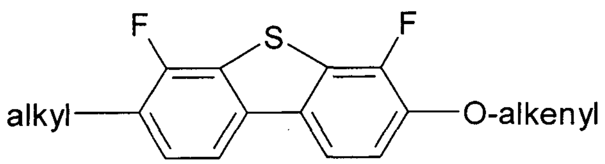

IA-7

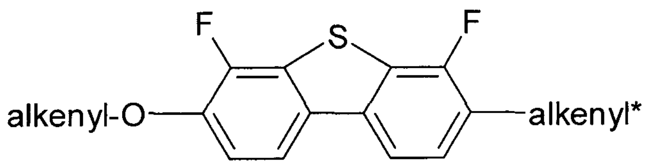

IA-8

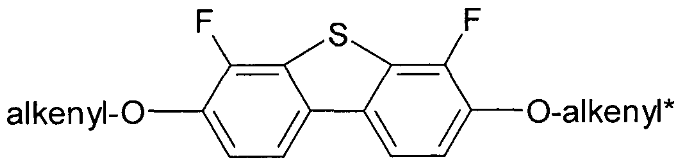

IA-9

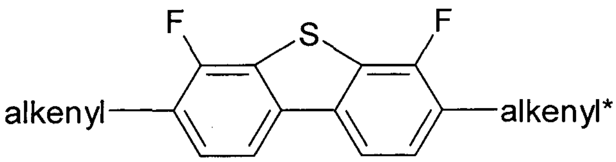

IA-10,

in which alkyl and alkyl* each, independently of one another, denote a straight-chain alkyl radical having 1-7 C atoms, alkenyl and alkenyl* each, independently of one another, denote a straight-chain alkenyl radical having 2-7 C atoms, alkoxy and alkoxy* each, independently of one another, denote a straight-chain alkoxy radical having 1-7 C atoms.

**7.** Compounds according to one or more of Claims 1 to 6, **characterised in that**

$R^1$ and $R^2$, independently of one another, denote an alkoxy or alkyl radical having 1 to 7 carbon atoms or an alkenyl radical having 2 to 7 carbon atoms.

**8.** Compounds according to one or more of Claims 1 to 7, **characterised in that**

$R^1$ and $R^2$ each denote an alkoxy group having 1 to 7 carbon atoms.

**9.** Compounds according to one or more of Claims 1 to 7, **characterised in that**

$$m + n = 1 \text{ or } 2, \text{ and}$$

or 2, and
the ring $A^1$ is in each case selected independently from the moieties

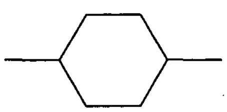
,
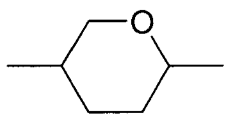
,
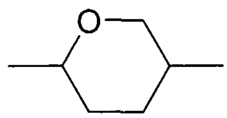
,

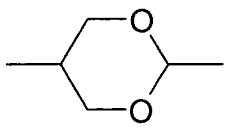
,
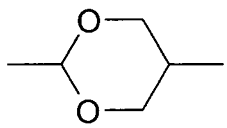
,
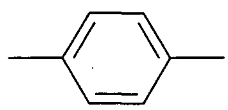
,

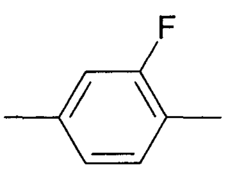
,
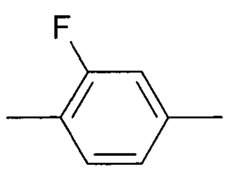
or
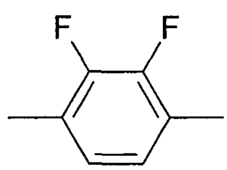
.

**10.** Use of one or more compounds according to one or more of Claims 1 to 9 in liquid-crystalline media.

**11.** Liquid-crystalline medium comprising at least two compounds, **characterised in that** it comprises at least one compound according to one or more of Claims 1 to 9.

**12.** Electro-optical display element containing a liquid-crystalline medium according to Claim 11.

**13.** Process for the preparation of compounds of the formula I according to one or more of Claims 1 to 9, **characterised in that** it includes a process step in which a compound of the formula (B)

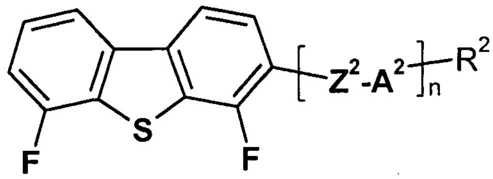

**(B)**

in which $Z^2$, $A^2$, n and $R^2$ independently are defined as in formula I according to one of Claims 1 to 9, is deprotonated at position 3 by means of a deprotonating reagent and converted into a compound of the formula (C)

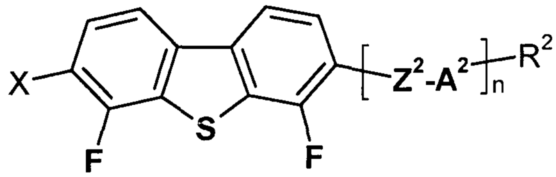

**(C)**

in which, independently,
X denotes $B(OR)_2$, $-C(OH)R_2$, -(CO)OH, -(CO)Cl, OH or

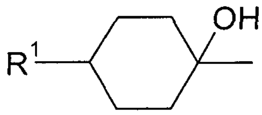

,

R independently denotes H or an alkyl radical having 1 to 14 C atoms, and
$Z^2$, $A^2$, n, $R^1$ and $R^2$ are defined as in formula I, and in further process steps is converted into a compound of the formula I.

## Revendications

**1.** Composés de la formule I :

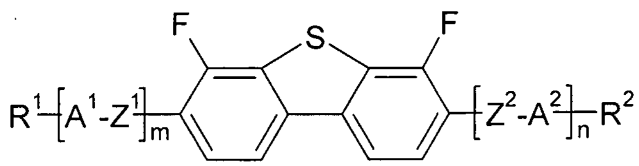

I

dans laquelle :

m et n sont chacun, indépendamment l'un de l'autre, 0, 1 ou 2,
$R^1$ et $R^2$ représentent, indépendamment l'un de l'autre, un radical alkyle ou alcoxy comportant 1 à 15 atome(s) de C, où, en outre, un ou plusieurs groupe(s) $CH_2$ dans ces radicaux peut/peuvent chacun être remplacé(s), indépendamment les uns des autres, par -C≡C-, $-CF_2O-$, $-OCF_2-$, -CH=CH-,

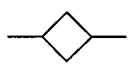 , 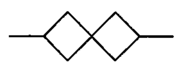 ,

-O-, -CO-O- ou -O-CO- de telle sorte que des atomes de O ne soient pas liés directement les uns aux autres, et où, en outre, un ou plusieurs atome(s) de H peut/peuvent être remplacé(s) par halogène,

$A^1$ et $A^2$ représentent, indépendamment l'un de l'autre, un radical choisi parmi les groupes qui suivent :

a) 1,4-phénylène, où, en outre, un ou deux groupe(s) CH peut/peuvent être remplacé(s) par N et où, en outre, un ou plusieurs atome(s) de H peut/peuvent être remplacé(s) par un groupe L,
b) le groupe constitué par trans-1,4-cyclohexylène et 1,4-cyclohexénylène, où, en outre, un ou plusieurs groupe(s) $CH_2$ non adjacents peut/peuvent être remplacé(s) par -O- et/ou -S- et où, en outre, un ou plusieurs atome(s) de H peut/peuvent être remplacé(s) par F ou Cl, et
c) le groupe constitué par tétrahydropyran-2,5-diyle, 1,3-dioxane-2,5-diyle, tétrahydrofuran-2,5-diyle, cyclobutane-1,3-diyle, pipéridine-1,4-diyle, thiophène-2,5-diyle et sélénophène-2,5-diyle, dont chacun peut également être monosubstitué ou polysubstitué par un groupe L,

L représente, pour chaque occurrence de manière indépendante, F, Cl, CN, SCN, $SF_5$ ou alkyle, alcoxy, alkylcarbonyle, alcoxycarbonyle, alkylcarbonyloxy ou alcoxycarbonyloxy en chaîne droite ou ramifié, dans chaque cas en option fluoré, comportant 1 à 12 atome(s) de C, et
$Z^1$ et $Z^2$ représentent, indépendamment l'un de l'autre, une liaison simple, $-CF_2O-$, $-OCF_2-$, $-CH_2O-$, $-OCH_2-$, -(CO)O-, -O(CO)-, $-(CH_2)_4-$, $-CH_2CH_2-$, $-CF_2-CF_2-$, $-CF_2-CH_2-$, $-CH_2-CF_2-$, -CH=CH-, -CF=CF-, -CF=CH-, -CH=CF-, $-(CH_2)_3O-$, $-O(CH_2)_3-$, -C≡C-, -O-, $-CH_2-$, $-(CH_2)_3-$ ou $-CF_2-$, de façon préférable une liaison simple.

2. Composés selon la revendication 1, **caractérisés en ce que** la somme m + n dans la formule I présente la valeur 0 ou 1.

3. Composés selon la revendication 1 ou 2, **caractérisés en ce que** :

$R^1$ et $R^2$ représentent, indépendamment l'un de l'autre, un radical alkyle ou un radical alcoxy non substitué comportant 1 à 15 atome(s) de carbone ou un radical alkényle, alkényloxy ou alkynyle comportant 2 à 15 atomes de C, dont chacun est en option monohalogéné ou polyhalogéné.

4. Composés selon une ou plusieurs des revendications 1 à 3, **caractérisés en ce que**, dans la formule I, m + n = 0.

5. Composés selon une ou plusieurs des revendications 1 à 4, **caractérisés en ce que** les composés de la formule I sont choisis parmi les sous-formules IA à IC :

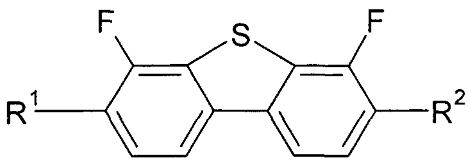

IA

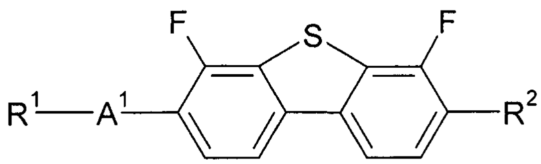

IB

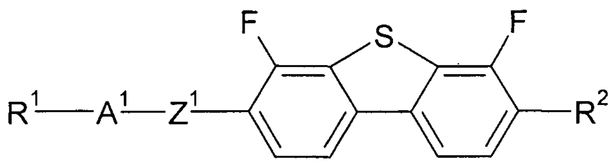

IC

dans lesquelles :

$R^1$, $R^2$, $A^1$ et $Z^1$ présentent les significations de la formule I selon la revendication 1.

6. Composés selon une ou plusieurs des revendications 1 à 5, **caractérisés en ce que** les composés sont choisis

parmi les composés des formules IA-1 à IA-10 :

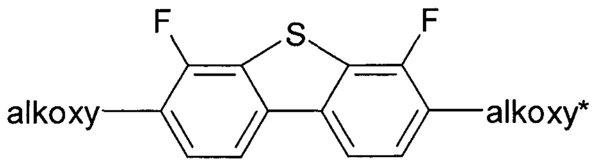

IA-1

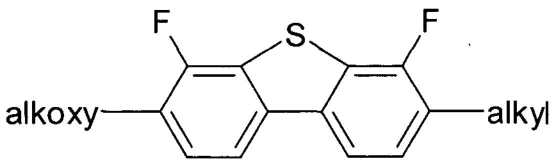

IA-2

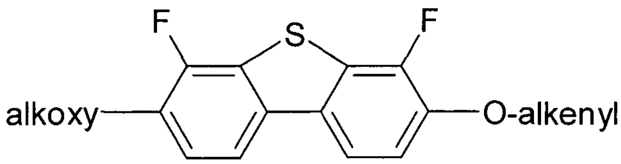

IA-3

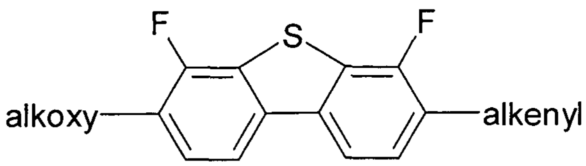

IA-4

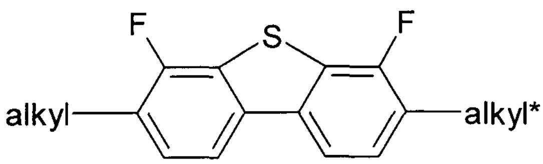

IA-5

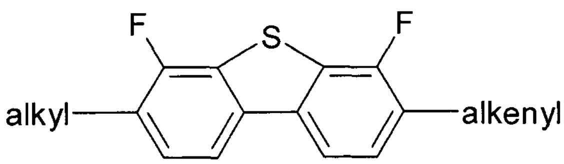

IA-6

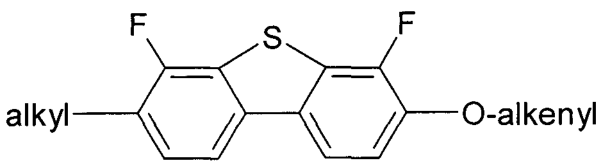

IA-7

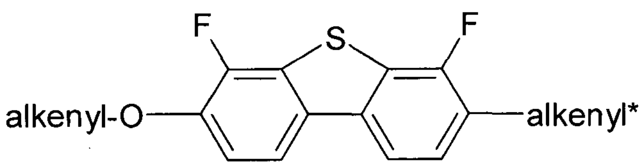

IA-8

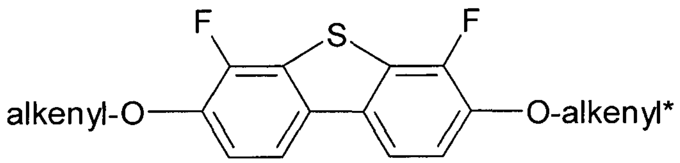 IA-9

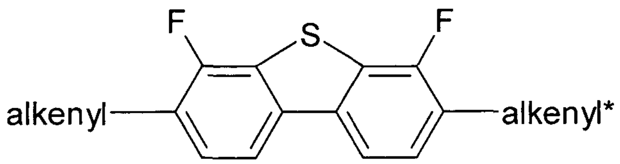 IA-10,

dans lesquelles alkyl et alkyl* représentent chacun, indépendamment l'un de l'autre, un radical alkyle en chaîne droite comportant 1-7 atome(s) de C, alkenyl et alkenyl* représentent chacun, indépendamment l'un de l'autre, un radical alkényle en chaîne droite comportant 2-7 atomes de C, alkoxy et alkoxy* représentent chacun, indépendamment l'un de l'autre, un radical alcoxy en chaîne droite comportant 1-7 atome(s) de C.

7. Composés selon une ou plusieurs des revendications 1 à 6, **caractérisés en ce que** :

$R^1$ et $R^2$ représentent, indépendamment l'un de l'autre, un radical alcoxy ou alkyle comportant 1 à 7 atome(s) de carbone ou un radical alkényle comportant 2 à 7 atomes de carbone.

8. Composés selon une ou plusieurs des revendications 1 à 7, **caractérisés en ce que** :

$R^1$ et $R^2$ représentent chacun un groupe alcoxy comportant 1 à 7 atome(s) de carbone.

9. Composés selon une ou plusieurs des revendications 1 à 7, **caractérisés en ce que** :

$$m + n = 1 \text{ ou } 2, \text{ et}$$

ou 2, et
le cycle $A^1$ est, dans chaque cas, choisi de manière indépendante parmi les moitiés :

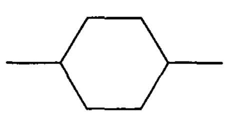 , 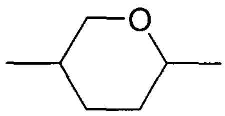 , 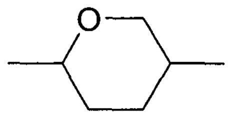 ,

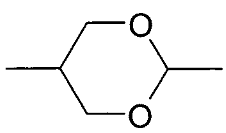 , 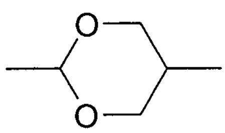 , 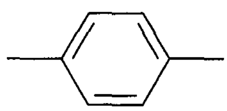 ,

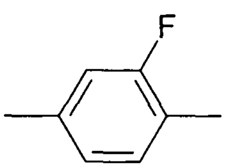 , 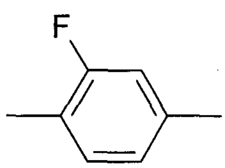 ou 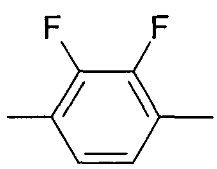 .

10. Utilisation d'un ou de plusieurs composé(s) selon une ou plusieurs des revendications 1 à 9 dans des milieux cristallins liquides.

**11.** Milieu cristallin liquide comprenant au moins deux composés, **caractérisé en ce qu'**il comprend au moins un composé selon une ou plusieurs des revendications 1 à 9.

**12.** Elément d'affichage électro-optique contenant un milieu cristallin liquide selon la revendication 11.

**13.** Procédé pour la préparation de composés de la formule I selon une ou plusieurs des revendications 1 à 9, **caractérisé en ce qu'**il inclut une étape de procédé selon laquelle un composé de la formule (B) :

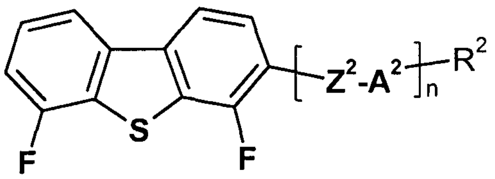

**(B)**

dans laquelle $Z^2$, $A^2$, n et $R^2$ sont définis, de manière indépendante, comme selon la formule I selon l'une des revendications 1 à 9, est déprotoné à la position 3 au moyen d'un réactif de déprotonation et est converti selon un composé de la formule (C) :

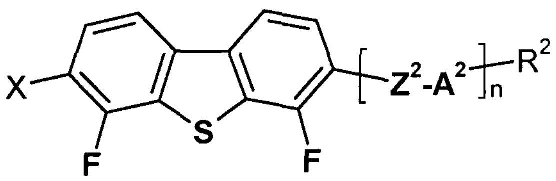

**(C)**

dans laquelle, de manière indépendante :

X représente $B(OR)_2$, $-C(OH)R_2$, -(CO)OH, -(CO)Cl, OH ou

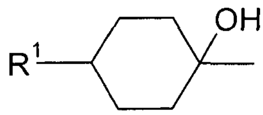

,

R représente de manière indépendante H ou un radical alkyle comportant 1 à 14 atome(s) de C, et $Z^2$, $A^2$, n, $R^1$ et $R^2$ sont comme défini selon la formule I, et selon d'autres étapes de procédé, est converti selon un composé de la formule I.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 02055463 A
- DE 102005012585
- EP 1752510 A


### In der Beschreibung aufgeführte Nicht-Patentliteratur


- **H. KELKER ; R. HATZ.** Handbook of Liquid Crystals. Verlag Chemie, 1980
- Merck Liquid Crystals - Licristal® - Physical Properties of Liquid Crystals - Description of the Measurements Methods. Merck KGaA, 1998